# EUROPEAN PATENT SPECIFICATION

(11) **EP 2 262 476 B1**
(45) Date of publication and mention of the grant of the patent: **06.06.2018**
(21) Application number: 08737584.6
(22) Date of filing: 05.05.2008
(51) Int. Cl.: A61K 9/08, A61K 45/06, A61P 27/02, A61K 31/415, A61K 31/498, A61K 39/395

(54) **DRUG DELIVERY TO THE ANTERIOR AND POSTERIOR SEGMENTS OF THE EYE USING EYE DROPS.**
ARZNEIMITTELABGABE AN DIE VORDEREN UND HINTEREN SEGMENTE DES AUGES MIT AUGENTROPFEN
ADMINISTRATION DE MÉDICAMENT AUX SEGMENTS ANTÉRIEUR ET POSTÉRIEUR DE L' IL À L'AIDE DE GOUTTES OPHTALMIQUES

(30) Priority: 11.03.2008 TN 08110
(43) Date of publication of application: 22.12.2010
(73) Proprietor: Rekik, Raouf, 1073 Tunis (TN)
(72) Inventor: Rekik, Raouf, 1073 Tunis (TN)
(74) Representative: Desaix, Anne
(86) International application number: PCT/IB2008/001102
(87) International publication number: WO 2009/112878

(56) References cited:
- WO-A-02/060339
- WO-A-03/103772
- WO-A-2005/021004
- WO-A-2008/080110
- WO-A2-00/76499
- WO-A2-2004/069181
- US-A1- 2004 071 761
- US-B1- 6 248 735
- ROTE LISTE SERVICE GMBH (ED.): "Rote Liste 2004" 2004, EDITIO CANTOR VERLAG , AULENDORF, GERMANY , XP002504094 product 67 077 "Dexa Biciron Augentropfen"

## Description

### Field

The present disclosure relates to a method to deliver drugs to the anterior and posterior segments of the eye and compositions thereof for such a delivery. More specifically the present disclosure relates to a kit or composition for use in a method wherein an alpha adrenergic agonist agent, a beta-blocking agent, a derivative or derivatives thereof, or mixture thereof is (are) administered with an effective amount of a drug that can treat chorio-retinal and/or optic nerve head disorders. Kits and compositions containing said alpha adrenergic agonist agent, beta-blocking agent, derivative or derivatives thereof, or mixture thereof and the drug are also disclosed.

### 2. Background and Related Prior Art

With the population living longer many disorders or diseases of the eyes have been appearing and are currently being treated by ophthalmologists. Over the past several years many advances in ophthalmic therapy have arisen in response to a rising need for improvement in this area.

Many ophthalmic disorders arise in the anterior and posterior segments of the eye. The anterior segment of the eye is the front third of the eye that includes the structures in front of the vitreous humor such as the cornea, iris, ciliary body and lens. Ophthalmic disorders associated with the anterior segment of the eye include glaucoma, cataract, congenital and developmental abnormalities, inflammatory and infectious diseases, hereditary and degenerative diseases and ocular manifestations of systemic diseases, tumors, injury and trauma.

The posterior segment of the eye is the back two-thirds of the eye that includes the anterior hyaloid membrane and all of the structures behind it such as the vitreous humor, the retina, the choroid and the optic nerve. Opthalmic disorders resulting in the posterior segment of the eye include age-related macular degeneration, diabetic retinopathy, retinal venous occlusions, retinal arterial occlusions, macular edema, post-operative inflammation, uveitis, retinitis, proliferative vitreoretinopathy, glaucoma neuropathy and high myopia macular degeneration.

Administering drugs for treatment of various disorders of the eye can be performed by a variety of methods known in the art such as by topical administration, administration of eye drops, intraocular injection and systemic administration.

However most of these methods have drawbacks. Eye drops and ointments that have been used for years are not always effective due to the eye's natural protective surface. Furthermore, by using this type of administration the drugs are rarely delivered to the posterior segments of the eye in proper quantities and hence cannot be used to treat various disorders and/or diseases of the chorio-retinal and/or optic nerve head disorders.

Systemic administration requires a very high dosage of drug and like topical administration very little of the administered compound enters the eye.

For treating disorders and/or diseases of the chorio-retina and/or optic nerve head the main type of administration utilized is injecting the various drugs directly into the eye, usually into the vitreous humor or subconjunctival injections. For example ranibizumab (Lucentis®) injection is known for treating wet age-related macular degeneration. Ranibizumab is a vascular endothelial growth factor (VEGF) antagonist that blocks abnormal blood vessel growth and leakage in the eye. It must be administered once a month by a physician.

Pegatanib (Macugen®) and bevacizumab (Avastin®) are other drugs for treating age-related macular degeneration. They must be delivered directly by injection through a needle into the eye, which requires medical assistance and is usually performed in a clinical setting.

In the cases of delivering the drugs by direct injection into the eye, usually only one eye is treated at a time to prevent complications and these techniques are invasive techniques that are often very discomforting to the patient. In some instances direct injection can lead to complications in the eyes that are even more serious than the disease or disorder itself.

Due to the membrane barriers of the cornea, conjunctiva and sclera and lachrymal drainage it is quite difficult to administer successfully drugs into the posterior segment of the eye other than by injection.

In general, drugs can enter the eye through three distinct routes; i.e., (1) the corneal route which is through the anterior chamber and then through the lens, the pupil or the iris; (2) the conjunctival route which either is directly across the sclera, choroid, choriocapillaries and retinal pigment epithelium to the retina or indirectly into the retrobulbar space and then the optic nerve head; and (3) from the systemic circulation after topical, parental, oral or intranasal or any other route that delivers the drug to the blood circulation.

The use of eye drops is generally a route of delivering drugs into the posterior segment of the eyes which is considered quite ineffective due to the lack of therapeutic amounts of the drugs that can be effective in this area of the eye. See, for example Myles et al Adv. Drug Del Rev. 57(14):2063-79). Due to this ineffectiveness, several attempts have been made to overcome this problem in the art.

Thus, U.S. Patent publication No. 2007/0020336 A1 describes the use of cyclodextrin nanotechnology for delivery to the posterior segment of the eye. This ophthalmic composition contains a drug, cyclodextrin and water in which about 0.1% to 90% (w/v) of the drug is dissolved in the solution and a solid phase consisting of particles which have a size of 10 nm to 1mm. This composition can be in the form of eye drops.

In yet another attempt to solve the problem with non-invasive posterior segment delivery of drugs U.S. publication No. 2005/0009910 A1 describes a method and composition which uses an effective concentration of an ester prodrug of the active drug. This composition is a sustained release composition in which a polymeric microparticle system enhances the release of the drug. The prodrug is administered via injection or implantation.

WO 2007/075720 A2 describes topical mecamylamine formulations for ocular administrations for the treatment of neovascularization, abnormal angiogenesis, vascular permeability or combinations thereof of posterior and/or anterior tissues and fluids of the eye.

WO 2004/069181 discloses a combination of Ramipril and timolol for the treatment of elevated intraocular blood pressure, said agents acting as aquaporin modulating agents and aqueous humor modulating agents.

WO 00/76499 discusses the treatment of various ophthalmic pathologies, such as glaucomatous neuropathy, degenerative choriopathy of strong myopia, age-related maculopathy, serious central chorioretinopahty, hereditary dystrophy of the retina, and retinal venous occlusions, through administration of Ramipril.

US 2004/071761 A1 discloses pharmaceutical formulations, for the treatment of ophtalmologic conditions, comprising an effective amount of a therapeutic compound, and means for prolonging the residence time of said therapeutic compound's contact with the eye. Appropriate means, which include phenylephrine, should act as vasoconstrictors.

Although the art has advanced quite rapidly in the last few years, there is still a need in this art to provide methods and compositions to deliver drugs to treat various ophthalmic disorders and/or diseases to the posterior segments of the eyes which drug is delivered through a non-invasive route.

Thus it is an object of the present invention to overcome the problems associated with drug delivery to the posterior segment of the eye known in the art.

Another object of the present disclosure is a method for delivering drugs to treat ophthalmic disorders or diseases that uses drugs known in the art to treat these disorders and/or diseases.

Yet another object of the present disclosure is to provide a non-invasive treatment for delivering drugs to treat ophthalmic disorders or diseases which is simple and less expensive.

Another object of the present disclosure is to provide an ophthalmic composition comprising an effective amount of a drug to treat the ophthalmic disorder and/or disease and an adrenergic agent selected from the group consisting of alpha adrenergic agonist agents, derivatives of the alpha adrenergic agonist agents, beta-blocking agents, derivatives of the beta-blocking agents and mixtures thereof, in an effective amount such that the drug used to treat the ophthalmic disorders and/or diseases of the eye is delivered to the posterior segment of the eye. Yet another aspect of the present disclosure concerns a method to deliver drugs to the chorio-retina and optic nerve head of the eye.

Use of an adrenergic agent selected from the group consisting of alpha adrenergic agonist agents, derivatives of the alpha adrenergic agonist agents, beta-blocking agents, derivatives of the beta-blocking agents and mixtures thereof, to deliver drugs to treat ophthalmic disorders and/or diseases is also part of the present disclosure. These and the objects achieved by the present invention are evidenced by the summary, description of the preferred embodiments and the claims.

The scope of the invention is defined by the claims. Any references in the description to methods of treatment refer to the kits or pharmaceutical compositions of the present invention for use in a method for treatment of the human or animal body by therapy, as claimed.

### Summary

The invention relates to a kit comprising or consisting of: a) ramipril and apraclonidine, or b) ramipril and brimonidine, or c) ramipril, neosynephrine, and timolol, in which apraclonidine, brimonidine, neosynephrine, and timolol can be administered either simultaneously or prior to Ramipril, as stated in claim 1. Other particular embodiments are the subject of claims 2 to 6.

The invention also relates to an ophthalmologic composition, as defined in claim 7.

The invention further relates to a kit or an ophthalmologic composition for use as a medicament, as defined in any one of claims 8 to 14.

### Brief Description of the Drawings

Fig. 1 is a photograph of an ocular fundus fluorescence of the right and left eyes of a patient who had apraclonidine and fluorescein administered in the right eye (A) and fluorescein in the left eye (B).
Fig. 2 is a photograph of an ocular fundus fluorescence of the right and left eyes of a patient who had neosynephrine and fluorescein administered in the right eye (A) and fluorescein in the left eye (B).
Fig. 3 is a photograph of an ocular fundus fluorescence of the right and left eyes of a patient who had fluorescein administered in the right eye (A) and timolol and fluorescein in the left eye (B).
Fig. 4 is a photograph of an ocular fundus fluorescence of the right and left eyes of a patient who had fluorescein administered in the right eye (A) and apraclonodin and fluorescein in the left eye (B).
Fig. 5 is a photograph of an ocular fundus fluorescence of the right and left eyes of a patient who had fluorescein administered in the right eye (A) and brimonidine (Alphagan) and fluorescein in the left eye (B).
Fig. 6 is a photograph of an ocular fundus and a fluoro-angiography of a diabetic patient (patient B.C.N.) who received topically a combination of apraclonidine and bevacizumab (Avastin®).
Fig. 7 is an optical coherence tomography scan (O.C.T.) of the same diabetic patient as the one of figure 6 (patient B.C.N) prior to treatment (A), and two months after treatment (B) with apraclonidine and bevacizumab (Avastin®).
Fig. 8 is a photograph of an ocular fundus and fluoro-angiography of a patient (patient G.N.) having non proliferative diabetic retinopathy before she receives topically a combination of a corticosteroid, a non-steroidal anti-inflammatory agent and apraclonidine.
Fig. 9 is an optical coherence tomography scan (O.C.T.) of the same patient as the one of figure 8 (patient G.N.) prior to treatment (A) and three months after treatment (B) with a combination of a corticosteroid, a non-steroidal anti-inflammatory agent and apraclonidine.
Fig. 10 is a photograph of an ocular fundus and fluoro-angiography of a patient (patient M.M.) having non proliferative diabetic retinopathy before she receives topically a combination of a corticosteroid and neosynephrine.
Fig. 11 is an optical coherence tomography scan (O.C.T.) of the same patient as the one of figure 10 (patient M.M.) prior to treatment (A) and three months after treatment (B) with a corticosteroid and neosynephrine.
Fig. 12 is a photograph of an ocular fundus and fluoro-angiography of a patient (patient H.M.) having proliferative diabetic retinopathy before (A) and 3 months after (B) he receives a combination of brimonidine and a non-steroidal anti-inflammatory agent.
Fig. 13 is an optical coherence tomography scan (O.C.T.) of the same patient as the one of figure 12 (patient H.M.) prior to treatment (A) and three months after treatment (B) with a combination of brimonidine and a non-steroidal anti-inflammatory agent.
Fig. 14 is a photograph of an ocular fundus and fluoro-angiography of a patient (patient N.B.) having non proliferative diabetic retinopathy before (A) and six months after (B) having received topically a combination of a corticosteroid, a non-steroidal anti-inflammatory agent and brimonidine.
Fig. 15 is an optical coherence tomography scan (O.C.T.) of the same patient as the one of figure 14 (patient N.B.) prior to treatment (A) and six months after treatment (B) with a combination of a corticosteroid, a non-steroidal anti-inflammatory agent and brimonidine.
Fig. 16 is a photograph of an ocular fundus and fluoro-angiography of a patient (patient M.R.) having diabetic retinopathy before he receives topically a combination of apraclonidine and bevacizumab (Avastin®).
Fig. 17 is an optical coherence tomography scan (O.C.T.) of the same patient as the one of figure 16 (patient M.R.) prior to treatment (A) and three months after treatment (B) with apraclonidine and bevacizumab (Avastin®).
Fig. 18 is a photograph of an ocular fundus and fluoro-angiography of a patient (patient N.B.) having non proliferative diabetic retinopathy and branched vein occlusion in the left eye before (A) and three months after (B) having received topically a combination of a corticosteroid, a non-steroidal anti-inflammatory and neosynephrine.
Fig. 19 is an optical coherence tomography scan (O.C.T.) of the same patient as the one of figure 18 (patient N.B.) prior to topical treatment (A) and three months after topical treatment (B) with a combination of a corticosteroid, a non-steroidal anti-inflammatory and neosynephrine.
Fig. 20 is a photograph of an ocular fundus and a fluoro-angiography of a patient (patient B.S.T.) having imminent central retinal vein occlusion in the left eye before (A) and two weeks after (B) having received topically a combination of a non-steroidal anti-inflammatory and apraclonidine.
Fig. 21 is an optical coherence tomography scan (O.C.T.) of the same patient as the one of figure 20 (patient B.S.T.) prior to (A) and eye two weeks after (B) topical treatment with a combination of a non-steroidal anti-inflammatory and apraclonidine.
Fig. 22 is a photograph of an ocular fundus and fluoro-angiography of a patient (patient A.H.) having central retinal vein occlusion in the left eye before (A) and three months after (B) having received topically a combination of a corticosteroid and a non-steroidal anti-inflammatory and neosynephrine.
Fig. 23 is an optical coherence tomography scan (O.C.T.) of the same patient as the one of figure 22 (patient A.H.) prior to topical treatment (A) and three months after topical treatment (B) with a combination of a corticosteroid, a non-steroidal anti-inflammatory and neosynephrine.
Fig. 24 is an optical coherence tomography scan (O.C.T.) of a patient (patient S.Z.) having branched retinal vein occlusion in the left eye prior to topical treatment (A) and three months after topical treatment (B) with a combination of ramipril and apracronidine.
Fig. 25 is a photograph of a fluoro-angiography (A) and an ocular fundus (B) and of a patient (patient D.M.H) having branched retinal occlusion in the right eye before he receives topically a combination of bevacizumab (Avastin®) and neosynephrine.
Fig. 26 is an optical coherence tomography scan (O.C.T.) of the same patient as the one of figure 25 (patient D.M.H.) prior to topical treatment (A) and one month after topical treatment (B) with a combination of bevacizumab (Avastin®) and neosynephrine.
Fig. 27 is a photograph of an ocular fundus (upper line) and a fluoro-angiography (lower line) of a patient (patient CH.A.) having a two-month history of decreased vision (because of age related macular degeneration) in the right eye before (A) and two months after (B) having received topically a combination of a corticosteroid, a non-steroidal anti-inflammatory agent and apraclonidine.
Fig. 28 is an optical coherence tomography scan (O.C.T.) of the same patient as the one of figure 27 (patient CH. A.) prior to topical treatment (A) and two months after topical treatment (B) with a combination of a corticosteroid, a non-steroidal anti-inflammatory agent and apraclonidine.
Fig. 29 is a photograph of an ocular fundus (upper line) and fluoro-angiography (lower line) of a patient (patient K.T.) having a three-month history of occult new vessel (age related macular degeneration) in both eyes before (A) and three months after (B) having received topically a combination of a corticosteroid, a non-steroidal anti-inflammatory agent and neosynephrine.
Fig. 30 is an optical coherence tomography scan (O.C.T.) of the same patient as the one of figure 29 (patient K.T.) prior to treatment (A) and three months after treatment (B) with a combination of a corticosteroid, a non-steroidal anti-inflammatory agent and neosynephrine.
Fig. 31 is a photograph of an ocular fundus (upper line) and fluoro-angiography (lower line) of a patient (patient D.F.) having a history of decreased vision in the left eye because of choroid occult new vessel (age related macular degeneration), before (A) and three months after (B) having received topically a combination of a corticosteroid, a non-steroidal anti-inflammatory agent and apraclonidine.
Fig. 32 is an optical coherence tomography scan (O.C.T.) of the same patient as the one of figure 31 (patient D.F.) prior to treatment (A) and three months after treatment (B) with a combination of a corticosteroid, a non-steroidal anti-inflammatory agent and apraclonidine.
Fig. 33 is an optical coherence tomography scan (O.C.T.) of a patient (patient D.M.) having a history of decreased vision in the right eye because of choroid occult new vessel (age related macular degeneration), prior to treatment (A) and one month after treatment (B) with a combination of bevacizumab (Avastin®) and apraclonidine.
Fig. 34 is a photograph of an ocular fundus and fluoro-angiography of a patient (patient B.S.S.) having complaints of decreased central vision in the right eye before treatment (A), two months after having received topically a combination of bevacizumab (Avastin®) and apraclonidine (B), four months after having stopped the topical treatment (C) and two weeks after readministration of bevacizumab and apraclonidine (D).
Fig. 35 is a vertical scan of an optical coherence tomography scan (O.C.T.) of a patient (patient G.C.) having complaints of decreased central vision in the right eye due to classic macular new vessels prior to treatment (A) and one week after treatment (B) with ramipril, timolol and neosynephrine.
Fig. 36 is a horizontal scan of an optical coherence tomography scan (O.C.T.) of the same patient as the one of figure 35 (patient G.C.) prior to treatment (A) and one week after treatment (B) with ramipril, timolol and neosynephrine.
Fig. 37 are results from field of vision tests from a patient (patient A.L.) having an open angle chronic glaucoma in the right eye prior to topical treatment (A) and two weeks after topical treatment (B) with ramipril and brimonidine.

### Detailed Description of the Preferred Embodiments of the Present Invention

As used herein the terminology "anterior segment of the eye" means the front third of the eye that includes the structures in front of the vitreous humor such as the cornea, iris, ciliary body and lens.

As used herein the terminology "posterior segments of the eye" means the back two-thirds of the eye that includes the anterior hyaloid membrane and the optical structures behind it such as the vitreous humor, retina, choroid, optic nerve and optic nerve head.

The term "optic nerve head" as used herein means the circular area in the back (posterior segment) of the eye where the optic nerve connects to the retina.

As used herein the term "chorio-retina" refers to the posterior segments of the eye in which the retina contacts the choroid, which is the middle membrane of the eye.

The terms "treating" and "treatment" mean that the eye disorder and/or eye disease is improved.

The term "eye disorders" encompasses changes in vision, in the appearance of the eye or having abnormal sensations in the eye. Eye disorders include optic nerve disorders, chorio-retinal disorders and trauma such as injuries to the eye.

As used herein, the term "eye diseases" means any disease of the eye such as of glaucomatous neuropathy, central serous chorio retinopathy, high myopia chorio-retinopathy, pigmentosa retinopathy, diabetic retinopathy, central retinal vein occlusion, branch retinal vein occlusion, presbyopia, age related vision degradation, central retinal artery occlusion, exsudative macular degeneration, uveitis, papillitis and endophthalmitis. This terminology also encompasses at least one of the above diseases and thus two or more diseases of the above diseases of the eye are also contemplated by this expression. In a particular embodiment, "eye diseases" refer to diseases that affect the posterior segment of the eye.

By "eye disorders and/or diseases of the eye", it is meant herein at least one eye disorder and/or disease of the eye; this term can encompass several (two, three or more than three) eye disorders and/or diseases of the eye.

The term "animal" includes mammalians, in particular humans and non human mammalians. The term "mammal" encompasses any of various warm-blooded vertebrate animals of the class Mammalia, including humans and non human mammalians, characterized by a covering of hair on the skin and, in the female, milk-producing mammary glands for nourishing the young.

As used herein "ophthalmologically acceptable carrier" means any carrier that has substantially no long term or permanent detrimental effect on the eye to which it is administered, in particular any carrier that can be placed in the eye and that does not cause eye irritation. Opthamologically acceptable carriers include water (distilled or deionized water), saline solutions, phosphate buffered saline solutions, and other aqueous media.

"Adrenergic agent" as disclosed herein encompasses an alpha adrenergic agonist agent, a derivative of an alpha adrenergic agonist agent, a beta-blocking agent, a derivative of a beta-blocking agents and mixtures thereof.

As used herein, an "alpha adrenergic agonist agent" is a drug which has effects similar to, or the same as, epinephrine (adrenaline) or which is susceptible to epinephrine, or similar substances, such as biological receptors. This term includes alpha 1 agonists, and alpha 2 agonists. Alpha 1 agonists stimulate phospholipase C activity in a human and an animal body, which results in vasoconstriction and mydriasis (excessive dilation of the pupil). Alpha 2 agonists are able to inhibit adenyl cyclase activity in a human and an animal body and are used notably as antihypertensives, sedatives, to reduce eye's aqueous humor secretions and to facilitate aqueous humor outflow via the uveoscleral route. Examples of alpha 1 agonist include neosynephrine. Examples of alpha 2 agonists include brimonidine, aprachlonidine and clonidine. Others alpha adrenergic agonist agents disclosed herein, which may be used in the disclosed methods and compounds include methoxamine, methylnorepinephrine, oxymetazoline, phenylephrine, neosynephrine pivalat, beta-methylepinephrine, guanfacine, guanabenz, guanoxabenz, guanethidine, tizanidine, and mixtures thereof. According to the invention, alpha adrenergic agonist agents encompass apraclonidine, brimonidine, or neosynephrine.

By "beta-blocking agent" (or beta-adrenergic antagonist agent) it is meant herein a drug which blocks the action of epinephrine (adrenaline) and/or norepinephrine (noradrenaline) in a human and an animal body. These compounds are used notably to lower intraocular tension and/or to reduce eye's aqueous humor secretions. This term encompasses antagonists of the beta 1, beta 2 and beta 3 adrenergic receptors. The beta-blocking agents disclosed herein, which may be used in the disclosed methods, compositions and kits include timolol, sotalol, propranolol, penbutolol, nadolol, metoprolol, labetalol, esmolol, carteolol, bisoprolol, betaxolol, atenolol, acebutolol, levobunolol, metipranolol and mixtures thereof. According to the invention, beta-blocking agent is timolol.

By "derivative of an alpha adrenergic agonist agent" according to the present disclosure, it is meant a compound obtained via a chemical modification of an alpha 1 agonist or an alpha 2 agonist, and which retains respectively the ability to stimulate phospholipase C activity or the ability to inhibit adenyl cyclase activity in an animal model such as a mouse, a rat or a monkey. Said derivatives are preferably amine-containing compounds, which more preferably have pKa's of greater than 7, preferably about 7.5 to 9. The alpha 1 or alpha 2 activity of a derivative of an adrenergic agonist agent can be shown for example, by applying, to one eye of a mouse, a rat or a monkey, few drops (one, two or three) of said derivative in solution in an ophthalmologically acceptable carrier, and applying, to the other eye of the same animal, the same volume of the ophthalmologically acceptable carrier alone, and comparing dilation of the pupil (in the case of an alpha 1 agonist derivative) or aqueous humor secretions (in the case of an alpha 2 agonist derivative) of both eyes. "Derivatives of an alpha adrenergic agonist agent" include imidazoline derivatives such as oxymetazoline, xylometazoline, tetrahydrozoline and the like. Also those derivatives defined in U.S. Patent Nos. 7,345,077 and 7,335,803 may also be used as derivatives in the methods, compositions and kits disclosed herein.

By "beta-blocking agent derivative" according to the present disclosure, it is meant a compound obtained via a chemical modification of a beta-blocking agent as defined above, and which retains the ability to lower intraocular tension and/or to reduce eye's aqueous humor secretions in an animal model such as a mouse, a rat or a monkey. These properties can be shown for example, by applying, to one eye of a mouse, a rat or a monkey, few drops (one, two or three) of said derivative in solution in an ophthalmologically acceptable carrier, and applying, to the other eye of the same animal, the same volume of the ophthalmologically acceptable carrier alone, and measuring and comparing intraocular tension and/or aqueous humor secretions of both eyes. Beta-blocking agent derivatives may include guaiacoxy propanolamine derivatives such as those described in U.S. Patent 5,804,603.

Thus, is part of a the present disclosure a method for delivering drugs to the posterior and anterior segments of the eyes (of one or both eye(s)) comprising contacting the surface of the eye with an effective amount of a drug for treating eye disorders and/or diseases of the eye and a physiologically acceptable amount of an adrenergic agent selected from the group consisting of alpha adrenergic agonist agents, derivatives of the alpha adrenergic agonist agents, beta-blocking agents, derivatives of the beta-blocking agents and mixtures thereof, in an opthalmologically acceptable carrier.

Accordingly, the methods, compositions and kits of the present disclosure provide for the treatment of many eye disorders and/or diseases of the eye such as glaucoma, glaucoma neuropathy, diabetic retinopathy, choroidal new vessels (age-related macular degeneration; high myopia; macular degeneration), uveitis (in particular anterior and/or posterior uveitis), eye infections, papillitis, endophthalmitis, optic nerve head inflammation, arterial or vein occlusion, central serous chorio-retinopathy (CSCR), pigmentosa retinopathy. The methods, compositions and kits disclosed herein can in particular be used for improving vision of one or both eyes, and more particularly for improving distance vision and/or near vision.

At least one eye disorder and/or disease of the eye can be treated with the methods and compositions disclosed herein and more than one or several eye disorders and/or diseases can also be treated.

The invention encompasses the embodiments defined in any one of claims 8 to 14. In the present disclosure, the drug to treat the disorders and/or diseases of the eyes can be administered topically in the form of suspensions, gels or ointments or in the form of eye drops or solutions along with a physiologically acceptable amount of an adrenergic agent selected from the group consisting of alpha adrenergic agonist agents, derivatives of the alpha adrenergic agonist agents, beta-blocking agents, derivatives of the beta-blocking agents and mixtures thereof.

In a particular embodiment, active compounds are administered in accordance with the present disclosure or invention to the eye admixed with an ophthalmically acceptable carrier. Any suitable, e.g., conventional, ophthalmically acceptable carrier as defined herein may be employed.

It may be desirable to formulate the drug for treating eye disorders and/or diseases of the eye and the adrenergic agent that can be used in the methods, the compositions and the kits of the present disclosure as topical agents to be instilled into the eye. Such formulations may contain the active ingredient in a concentration range of approximately 0.01% to 20% weight by volume (w/v), preferably from 0.05% to 10% (w/v), and more preferably from 0.5% to 3% (w/v). The composition itself may include, in addition to the active ingredient, excipients which are per se well know in the art for preparing ophthalmic compositions, particularly ophthalmic solutions.

The ophthalmic compositions (solutions or other formulations) that contain the adrenergic agent(s) and/or the drug(s) for treating disorders and/or diseases of the eyes may be administered to the mammalian eye as often as necessary to obtain an improvement of the eye disorder and/or eye disease. Those skilled in the art will recognize that the frequency of administration and duration of treatment depends on the precise nature of the active ingredient(s) and its concentration in the ophthalmic formulation, and various factors such as the type and severity of the eye disorder and/or eye disease, the age and weight of the patient, the patient's general physical condition and the cause of the eye disorder and/or eye disease. Within these guidelines it is contemplated that the ophthalmic formulations (preferably ophthalmic solutions) of the present disclosure will be administered topically to the mammalian eye approximately once, twice or three times daily. The duration of treatment administered in accordance with the present disclosure may range, for example, from few weeks (at least one week) to few months (at least one month), in particular from 1 week to 6 months, preferably at least 2 weeks and less than 4 months and more preferably at least 3 weeks and less than 3 months. However, a prolonged treatment may be required. In particular, the treatment may last for life, for example in case of recurrence of the eye disorder and/or eye disease.

In a particular embodiment of the disclosure or invention, the physiologically acceptable amount of the adrenergic agent(s) as defined herein is administered prior to administering the effective amount of the drug(s) for treating disorders and/or diseases of the eyes; hence, in this case, the adrenergic agent(s) contact the eye prior to the drug(s) for treating disorders and/or diseases of the eyes.

The alpha adrenergic agonist agents that can be used in the methods, the compositions and the kits disclosed herein can be selected from the group comprising or consisting of methoxamine, methylnorepinephrine, oxymetazoline, phenylephrine, neosynephrine, in particular neosynephrine pivalat, beta-methylepinephrine, brimonidine, apraclonidine, clonidine, guanfacine, guanabenz, guanoxabenz, guanethidine, tizanidine, and mixtures thereof. The alpha adrenergic agonist agents that can be used in the present invention encompass apraclonidine, brimonidine, or neosynephrine.

The beta-blocking agents that can be used in the methods, the compositions and the kits disclosed herein can be selected from the group comprising or consisting of timolol, sotalol, propranolol, penbutolol, nadolol, metoprolol, labetalol, esmolol, carteolol, bisoprolol, betaxolol, bisoprolol, atenolol, acebutolol, levobunolol, metipranolol and mixtures thereof. The beta-blocking agent that can be used in the present invention is timolol.

The actual amount of the adrenergic agent(s) and the drug(s) for treating eye disorders and/or diseases of the eye to be administered in any given case will be determined by a physician taking into account the relevant circumstances, such as the type and severity of the eye disorder and/or eye disease, the age and weight of the patient, the patient's general physical condition and the cause of the eye disorder and/or eye disease.

By way of example, physiologically acceptable amounts of the alpha adrenergic agonist agent, the beta-blocking agent, the derivative(s) of an alpha adrenergic agonist agent, the derivative(s) of a beta-blocking agent and mixtures thereof that are generally administered to a person or an animal in need thereof are ranging from 0.01% to 20% (w/v), preferably from 0.1% to 15% (w/v) and more preferably from 0.1% to 3% (w/v), for example from 0.1% to 2% (w/v) or 0.2% to 1% (w/v) if eyedrops are used and from 0.1% to 2% (w/v) in the case of topical administration.

The effective amount of a drug for treating eye disorders and/or diseases of the eye is generally administered to a person or an animal in need thereof in a concentration ranging from 0.001 to 15% (w/v), preferably from 0.05 to 10% (w/v), and more preferably from 0.1 to 3% (w/v).

The substances or drugs used for treating disorders of the eyes and/or eye diseases can be selected from the group of calcium antagonists, angiotensin converting enzyme inhibitors, nitrates or nitric oxide generators, beta adrenergic agonists, antioxidants and radical scavengers, dopaminergic and serotoninergic agents, monoamine oxidase inhibitors, anti-inflammatory agents, growth factors, neuroprotective agents, growth factor vasoactive agents, neuropeptides, anti-inflammatory mediators, anti-infective agents, anti-ischemic association agents, non-steroidal anti-inflammatory agents, anti-growth factor agents, and mixtures thereof. According to the invention, the drug used for treating disorders of the eyes and/or eye diseases is the angiotensin converting enzyme inhibitor that is Ramipril.

The drugs or combinations of drugs according to the present disclosure or invention can be administered at room temperature.

Examples of calcium antagonists that can be used in the methods, the compositions and the kits disclosed herein can be selected from the group comprising verapamil, nifedipine, nimadipine, diltiazem, nicardipine, felodipine, amlodipine, isradipine and mixtures thereof.

Examples of angiotensin converting enzyme inhibitors that can be used in the methods, the compositions and the kits disclosed herein are selected from the group comprising captopril, enalapril, usinopril, ramipril, kinapril, benazepril, cilazapril and mixtures thereof.

Nitrates, isorbide dinitrate, isorbide mononitrate, linsidomine and mixtures thereof are examples of nitrates or nitric oxide generators that can be used in the methods, the compositions and the kits of the present disclosure.
Beta adrenergic agonists that can be used in the methods, the compositions and the kits of the present disclosure can be selected from the group comprising salbutamol, terbutalin, isoprenalin and mixtures thereof while antioxidants and radical scavengers that can be used in the present disclosure can be selected from the group comprising ascorbic acid, glutathione catalases and their derivatives and mixtures thereof.

Dopaminergic and serotoninergic agent that can be used in the methods, the compositions and the kits of the present disclosure can be selected from the group comprising: levodopa, amantadine, bromocriptine, serotonine and mixtures thereof.

Amitryptiline, nortryptyline, selegiline and mixtures thereof are monoamine oxidase inhibitors that can be used in the methods, the compositions and the kits of the present disclosure.

Examples of anti-inflammatory agents that can be used in the methods, the compositions and the kits of the present disclosure are non-steroidal anti-inflammatory agents or steroidal anti-inflammatory agents, in particular corticosteroids, or mixtures thereof.

Examples of non-steroidal anti-inflammatory drugs that can be used in the methods, the compositions and the kits of the present disclosure can be selected from the group comprising or consisting of aspirin, arylalkanoic acids such as bromfenac, indometacin, oxameticin, 2-arylpropionic acids such as fenbufen, pirprofen, ketoprofen, ibuprofen, oxaprozin, and ketorolac, femamic acids, pyrazolidine derivatives such as clofezonem kebuzone and phenazone, ocicams such as droxicam and meloxicam, and COX-2 inhibitors, such as celecoxib and rofecoxib.

Examples of corticosteroids that can be used in the methods, the compositions and the kits of the present disclosure can be selected from the group consisting of cortisone, hydrocortisone, deltacortisone or prednisolone, prednisone, deltahydrocortisone or prednisolone, methylprednisolone or medrocortisone, fluorohydrocortisone or fluorocortisone, fluoromethylprednisolone or dexamethazone, fluoromethyldeltahydrocortisone or betamethazone and paramethazone.

Growth factors such as nerve growth factors (NGF), epidermal growth factor (EGF), platelet derived growth factor (PDGF), transforming growth factor (TGF) and mixtures thereof can be used in the methods, the compositions and the kits of the present disclosure.

Anti-growth factor agents that can be used in the methods, the compositions and the kits of the present disclosure include anti-vascular endothelial growth factor (anti-VEGF) agents, anti-insulin like growth factor (anti-IGF) agents, anti-fibroblast growth factor (anti-FGF) agents, anti-platelet derived growth factor (anti-PDGF) agents, anti-placenta growth factor agents and mixtures thereof.

Anti-VEGF agents that can be used in the methods, the compositions and the kits of the present disclosure can be selected from the group comprising bevacizumab (Avastin®), ranibizumab (Lucentis®), pegaptanib (Macugen®), and mixtures thereof.

Anti-inflammatory mediators that can be used in the methods, the compositions and the kits of the present disclosure can be selected from the group comprising cytokines, bradikinine, histamine, serotonin, thrombin, ADP, acethylcholine, adrenalin and derivatives and mixtures thereof.

Anti-infective agents that can be used in the methods, the compositions and the kits of the present disclosure can be selected from the group comprising antibiotics, antifungal agents, antiviral agents and mixtures thereof.

Anti-ischemic association compounds selected from the group comprising angiotensin converting enzyme inhibitors, non-steroidal anti-inflammatory agents and mixtures thereof, can also be used in the methods, the compositions and the kits of the present disclosure.

In a particular embodiment, the drugs (i.e., the adrenergic agent(s) and the drug(s) to for treating disorders and/or diseases of the eyes) are delivered to the posterior segment of the eye. Said drugs can be in particular delivered to the chorio-retina and optic nerve head of the eyes.

In another aspect, the present disclosure relates to a method of treating eye disorders and/or diseases of the eye by delivering drugs to the chorio-retina and optic nerve head of an eye comprising administering to a person or an animal in need of such treatment
- an effective amount of a drug for treatment of the chorio-retina and/or optic nerve head and
- a physiologically acceptable amount of an adrenergic agent selected from the group consisting of alpha adrenergic agonist agents, derivatives of the alpha adrenergic agonist agents, beta-blocking agents, derivatives of the beta-blocking agents and mixtures thereof.

As set forth above the effective amount of the drug for treating chorio-retina and optic nerve head can be selected among the group comprising calcium antagonists, angiotensin converting enzyme inhibitors, nitrates or nitric oxide generators, beta-adrenergic agonists, antioxidants and radical scavengers, dopaminergic and serotoninergic agents, monoamine oxidase inhibitors, anti-inflammatory agents, growth factors, neuropeptides, anti-inflammatory mediators, anti-infective agents, non-steroidal anti-inflammatory agents, anti-ischemic association agents (non-steroidal anti-inflammatory agents and angiotensin converting enzyme inhibitors), neuroprotective agents, growth factor vasoactive agents, anti-growth factor agents, in particular anti-vascular endothelial growth factor (anti-VEGF) agents, anti-insulin like growth factor (anti-IGF) agents, anti-fibroblast growth factor (anti-FGF) agents, anti-platelet derived growth factor (anti-PDGF) agents, anti-placenta growth factor agents, and mixtures thereof. The specific drugs and amounts that can also be used in this method are set forth above. According to the invention, the drug used for treating disorders of the eyes and/or eye diseases is the angiotensin converting enzyme inhibitor that is Ramipril.

The alpha adrenergic agonist agents that can be used in the methods, the compositions and the kits of the present disclosure or invention are as defined above.

The beta-blocking agents that can be used in the methods, the compositions and the kits of the present disclosure or invention are as defined above.

The effective amount of a drug for treatment of the chorio-retina and/or optic nerve head is administered in a concentration ranging from 0.001 to 15% (w/v), preferably from 0.05 to 10% (w/v), and more preferably from 0.1 to 3% (w/v).

The physiologically acceptable amounts of the alpha adrenergic agonist agent, the beta-blocking agent, the derivative of an alpha adrenergic agonist agent, the derivative of a beta-blocking agent and mixtures thereof that are generally administered according to the present disclosure or invention are ranging from 0.01% to 20% (w/v), preferably from 0.1% to 15% (w/v), and more preferably from 0.1% to 3% (w/v), for example from 0.1% to 2% (w/v) or 0.2% to 1% (w/v) if eyedrops are used and from 0.1% to 2% (w/v) in the case of topical administration. More specifically, the physiologically acceptable amounts of alpha adrenergic agonist agent(s) or derivatives thereof that are generally administered vary from 0.01% to 20% (w/v), preferably from 0.1 to 3% (w/v) while the physiologically acceptable amounts of beta-blocking agent(s) or derivatives thereof that are generally administered vary from 0.05% to 2% (w/v), preferably 0.1% to 1% (w/v), and more preferably from 0.1 % to 0.5% (w/v).

In the method for treating chorio-retina and optic nerve head of the present disclosure, or kit or composition for use according to the invention, the drug(s) and the physiologically acceptable amounts of an adrenergic agent can be administered simultaneously or said adrenergic agent can be administered prior to the drug(s) which are used to treat the at least one eye disorder and/or eye disease.

If said adrenergic agent selected from the group consisting of alpha adrenergic agonist agents, derivatives of the alpha adrenergic agonist agents, beta-blocking agents, derivatives of the beta-blocking agents and mixtures thereof according to the present disclosure or invention is administered before the drug(s), usually it is administered from 1 second to 3 hours, preferably from 5 to 60 minutes, prior to the administration of the treating drug(s).

In yet another embodiment, the present disclosure provides a composition comprising, consisting or consisting essentially of
a) an adrenergic agent selected from the group consisting of alpha adrenergic agonist agents, derivatives of the alpha adrenergic agonist agents, beta-blocking agents, derivatives of the beta-blocking agents and mixtures thereof, and
b) a drug that treats eye disorders and/or diseases of the eye.

Said drug for treating eye disorders and/or diseases of the eye can be selected among the group comprising calcium antagonists, angiotensin converting enzyme inhibitors, nitrates or nitric oxide generators, beta-adrenergic agonists, antioxidants and radical scavengers, dopaminergic and serotoninergic agents, monoamine oxidase inhibitors, anti-inflammatory agents, growth factors, neuropeptides, anti-inflammatory mediators, anti-infective agents, non-steroidal anti-inflammatory agents, anti-ischemic association agents (non-steroidal anti-inflammatory agents and angiotensin converting enzyme inhibitors), anti-growth factor agents, in particular anti-vascular endothelial growth factor (anti-VEGF) agents, anti-insulin like growth factor (anti-IGF) agents, anti-fibroblast growth factor (anti-FGF) agents, anti-platelet derived growth factor (anti-PDGF) agents, anti-placenta growth factor agents, and mixtures thereof.

In a particular aspect, said composition can comprise both (i) an alpha adrenergic agonist agents or a derivative thereof and (ii) a beta-blocking agent, or a derivative thereof.

In a particular aspect, said drug for treating eye disorders and/or diseases of the eye can be selected from the group consisting of angiotensin converting enzyme inhibitors, non-steroidal anti-inflammatory agents, anti-growth factor agents, steroidal anti-inflammatory agents, in particular corticosteroids, and mixtures thereof. Said drug(s) are as set forth above.

In another particular aspect, said drug for treating eye disorders and/or diseases of the eye can be an anti-growth factor agent as defined herein, in particular an anti-vascular endothelial growth factor agent (anti-VEGF agent), a corticosteroid as set forth above or mixtures thereof.

In another particular aspect, said drug for treating eye disorders and/or diseases of the eye is an angiotensin converting enzyme inhibitor and/or a non-steroidal inflammatory agent (anti-ischemic complex). In this case, the adrenergic agent is generally selected from the group consisting of alpha adrenergic agonist agents, derivatives of the alpha adrenergic agonist agents and mixtures thereof. Said drug(s) are as set forth above.

In another particular aspect, said drug for treating disorders and/or diseases of the eyes is a corticosteroid and/or an anti-VEGF agent. Said drug(s) are as set forth above.

The invention more specifically provides a composition as defined in claim 7.

The compositions of the invention can be used as a medicament. In particular, said composition are suitable for the treatment and in particular the topical treatment of a disorder and/or a disease of the eye selected from the group comprising or consisting of diabetic retinopathy, retinal artery and vein occlusion, age related vision degradation (near and far visual acuity; visual field), macular oedema, central serious chorio-retinopathy, exsudative macular degeneration (age related macular degeneration, high myopia; macular degeneration) uveitis, papillitis, glaucomaneuropathy. The invention more specifically provides a kit or a composition for use as defined in the claims.

The alpha adrenergic agonist agents that can be used in the methods, the compositions and the kits of the present disclosure or invention are as defined above.

The beta-blocking agents that can be used in the methods, the compositions and the kits of the present disclosure or invention are as defined above

In such a composition as disclosed the angiotensin converting enzyme inhibitors that can be used can be selected from the group comprising or consisting of captopril, enalapril, usinopril, ramipril, kinapril, benazepril, cilazapril and mixtures thereof.

The non-steroidal anti-inflammatory agents that can be used in the composition can be selected from the group comprising or consisting of aspirin, arylalkanoic acids such as bromfenac, indometacin, oxameticin, 2-arylpropionic acids such as fenbufen, pirprofen, ketoprofen, ibuprofen, oxaprozin, and ketorolac, femamic acids, pyrazolidine derivatives such as clofezonem kebuzone and phenazone, ocicams such as droxicam and meloxicam, and COX-2 inhibitors, such as celecoxib and rofecoxib.

In the compositions of the present disclosure or invention, the adrenergic agent is generally present in amounts ranging from 0.01% to 20% (w/v), preferably from 0.1% to 15% (w/v), and more preferably from 0.1% to 3% (w/v), for example from 0.1% to 3% (w/v), 0.2% to 2% (w/v) or 0.1% to 0.5% (w/v). More specifically, the amounts of alpha adrenergic agonist agent(s) or derivatives thereof that are present in the compositions of the disclosure or invention generally vary from 0.01% to 20% (w/v), preferably from 0.1% to 3% (w/v) while the amounts of beta-blocking agent(s) or derivatives thereof that are present in the compositions of the disclosure or invention generally vary from in the amounts of 0.05% to 2% (w/v), preferably 0.1% to 1% (w/v), and more preferably from 0.1% to 0.5% (w/v). The drug for treating eye disorders and/or diseases of the eye is generally present in amounts ranging from 0.001 % to 15% (w/v), preferably from 0.05% to 10% (w/v), and more preferably from 0.1% to 3% (w/v).

In yet another aspect the present disclosure provides a composition for the topical treatment of diabetic retinopathy macular oedema, exudative macular degeneration, central retinal vein occlusion or branch retinal vein occlusion, uveitis, papillitis, or endophtalmitis comprising, consisting or consisting essentially of
a) an adrenergic agent selected from the group consisting of alpha adrenergic agonist agents, derivatives of the alpha adrenergic agonist agents, beta-blocking agents, derivatives of the beta-blocking agents and mixtures thereof, and
b) angiotensin converting enzyme inhibitor(s) and/or non steroidal anti-inflammatory agent(s) and/or steroidal anti-inflammatory agent(s), in particular corticosteroid(s), or mixtures thereof.

The adrenergic agent, angiotensin converting enzyme inhibitor(s), non steroidal anti-inflammatory agent(s) and corticosteroid(s) used in this composition can be any drug set forth above with respect to the other methods and compositions.

The amounts of alpha adrenergic agent(s), beta-blocking agent(s), derivative(s) thereof or mixtures thereof that can be used are as set forth above.

The amounts of angiotensin converting enzyme inhibitor(s), non steroidal anti-inflammatory agent(s) and/or corticosteroid(s) that can be used are generally ranging from 0.001 % to 15% (w/v), preferably from 0.05% to 10% (w/v), and more preferably from 0.1% to 3% (w/v).

In yet another aspect the present disclosure provides a composition for the topical treatment of age related vision degradation and presbyopia comprising or consisting of:
a) an alpha adrenergic agonist agent and/or a derivative of an alpha adrenergic agonist agent and
b) an angiotensin converting enzyme inhibitor and/or a non steroidal anti-inflammatory agent and/or an anti vascular endothelial growth factor agent (anti-VEGF agent) selected from the group comprising bevacizumab (Avastin®), ranibizumab (lucentis®), pegaptanib (Macugen®) and mixtures thereof.

The alpha adrenergic agonist agent, angiotensin converting enzyme inhibitor and non steroidal anti-inflammatory agent and corticosteroid(s) used in this composition can be any drug set forth above with respect to the other methods and compositions.

The amounts of alpha adrenergic agent(s), derivative(s) thereof or mixtures thereof that can be used are as set forth above.

The amounts of angiotensin converting enzyme inhibitor(s), non steroidal anti-inflammatory agent(s) and/or anti-VEGF agent(s) that can be used are generally ranging from 0.001% to 15% (w/v), preferably from 0.05% to 10% (w/v), and more preferably from 0.1 % to 3% (w/v).

In yet another aspect, the present disclosure provides a method for increasing the transfer of a drug into the eye orbit, the posterior sclera and then into chorioretina and optic nerve head to treat disorders and/or diseases of the eye comprising contacting the surface of an eye or both eyes with
a) a physiologically acceptable adrenergic agent selected from the group consisting of alpha adrenergic agonist agents, derivatives of the alpha adrenergic agonist agents, beta-blocking agents, derivatives of the beta-blocking agents and mixtures thereof, and
b) a pharmaceutically acceptable amount of a second drug that treats disorders and/or diseases of the eyes.

As set forth above the alpha adrenergic agonist agents that can be used in the methods, the compositions and the kits of the present disclosure are as defined above

The beta-blocking agents that can be used in the methods, the compositions and the kits of the present disclosure are as defined above

The amounts of alpha adrenergic agent(s), beta-blocking agent(s), derivative(s) thereof or mixtures thereof that can be used are as set forth above.

The pharmaceutically acceptable amount of a second drug that treats disorders and/or diseases of the eyes that can be used is as set forth above.

An adrenergic agent for the transfer, to the posterior segment of the eyes, of a second drug that treats disorders and/or diseases of the eyes, for the treatment of diseases and/or disorders of the eye is another aspect of the present disclosure. Said adrenergic agent is selected from the group consisting of alpha adrenergic agonist agents, derivatives of the alpha adrenergic agonist agents, beta-blocking agents, derivatives of the beta-blocking agents and mixtures thereof.

As set forth above the alpha adrenergic agonist agents that can be used in the methods, the compositions and the kits of the present disclosure are as defined above

The beta-blocking agents that can be used in the methods, the compositions and the kits of the present disclosure are as defined above

The amounts of alpha adrenergic agent(s), beta-blocking agent(s), derivative(s) thereof or mixtures thereof that can be used are as set forth above.

The second drug that treats disorders and/or diseases of the eyes that can be used in the methods, the compositions and the kits of the present disclosure can be selected from the group comprising or consisting of a calcium antagonists, nitrates or nitric oxide generators, beta adrenergic agonists, antioxidants and radical scavengers, dopaminergic and serotoninergic agents, monoamine oxidase inhibitors, anti-inflammatory agents, growth factors, neuropeptides, anti-inflammatory mediators, anti-infective agents, anti-ischemic association agents (non-steroidal anti-inflammatory agents and angiotensin converting enzyme inhibitors), anti-growth factor agents, in particular anti-vascular endothelial growth factor (anti-VEGF) agents, anti-insulin like growth factor (anti-IGF) agents, anti-fibroblast growth factor (anti-FGF) agents, anti-platelet derived growth factor (anti-PDGF) agents, anti-placenta growth factor agents, and mixtures thereof. The specific drugs that can also be used in this method are set forth above. The specific drugs that are used to treat eye disorders and/or diseases can be any drug set forth above with respect to the other methods.

The amounts of alpha adrenergic agent(s), beta-blocking agent(s), derivative(s) thereof or mixtures thereof that can be used are as set forth above.

The pharmaceutically acceptable amount of the second drug depends upon which drug is being used. Examples of pharmaceutically acceptable amounts include amounts ranging from 0.1 to 15% (w/v), preferably from 0.5 to 10%, and more preferably from 0.5 to 3% (w/v).

The present disclosure also provides use of
a) an adrenergic agent selected from the group consisting of alpha adrenergic agonist agents, derivatives of the alpha adrenergic agonist agents, beta-blocking agents, derivatives of the beta-blocking agents and mixtures thereof, and
b) a pharmaceutically acceptable amount of a second drug that treats disorders and/or diseases of the eyes,
for the manufacture of a medicament to treat disorders and/or diseases of the eye.

As set forth above the alpha adrenergic agonist agents that can be used in the methods, the compositions and the kits of the present disclosure are as defined above

The beta-blocking agents that can be used in the methods, the compositions and the kits of the present disclosure are as defined above

The amounts of adrenergic agents that are present in this medicament are as set forth above.

The pharmaceutically acceptable amount of a second drug that treats disorders and/or diseases of the eyes that can be used is as set forth above.

In yet another embodiment the present disclosure provides a kit comprising, consisting or consisting essentially of:
- (a) an adrenergic agent selected from the group consisting of alpha adrenergic agonist agents, derivatives of the alpha adrenergic agonist agents, beta-blocking agents, derivatives of the beta-blocking agents and mixtures thereof, or a composition comprising, consisting or consisting essentially of said adrenergic agent,
   and
- (b) a drug that treats disorders and/or diseases of the eyes.

The alpha adrenergic agents, beta-blocking agents, and derivatives thereof, as well as the drug for treating disorders and/or diseases of the eyes are as set forth above.

The amounts of said adrenergic agent and said drug for treating disorders and/or diseases of the eyes that can be used are as set forth above.

The kit of invention is as defined in any one of claims 1 to 6.

These kits can be used in the methods of the present disclosure and according to the invention, for use in treating chorio-retinal and/or optic nerve head disorders in a person or an animal.

In a particular aspect, said kits comprise both (i) an alpha adrenergic agonist agents or a derivative thereof and (ii) a beta-blocking agent, or a derivative thereof. According to the invention, said ingredients are as defined in claim 1.

In a particular aspect, said drug for treating disorders and/or diseases of the eyes can be selected from the group consisting of angiotensin converting enzyme inhibitors, non-steroidal anti-inflammatory agents, anti-growth factor agents, steroidal anti-inflammatory agents, in particular corticosteroids, and mixtures thereof. These drugs are as set forth above. According to the invention, said drug for treating disorders and/or diseases of the eyes is Ramipril.

The invention will now be illustrated by the following description of clinical examples. Further characteristics of the invention will become clear from the following clinical observations. Clinical observations provided in Examples 2 and 4 are provided as comparative examples only. Clinical observations provided in examples 3 and 5 are only in line with the present invention as far as they relate to ramipril as the ophthalmic drug, while clinical observations with other ophthalmic drugs are comparative examples.

### EXAMPLES

### BACKGROUND AND METHODS USED

There are two general pathways whereby a drug can reach the posterior segment of the eye from an eye drop:
*1. **Corneal:*** into the anterior chamber, and then through the lens, the pupil or the iris.
*2.* ***Conjunctival:*** either directly across the sclera, choroid, choriocapillaris and retinal pigment epithelium to the retina, or indirectly into the retrobulbar space and then the ONH (optic nerve head).

There is evidence that mechanically blocking of the corneal surface has little effect on drug penetration into the posterior tissues, which suggests that the conjunctival route is the more important for drug delivery.

When a large drop is allowed to flood the interpalpebal space, the fluid could fall under gravity and distend the cul-de-sac. In that case, the drug would have the opportunity to penetrate into the posterior sclera and orbit. The penetration from a drop to the posterior segment is increased by using an alpha adrenergic agonist agent or a beta-blocking agent as the drug carrier.

Alpha adrenergic agonist agents or beta-blocking agents or both enhance the transfer of drugs to the chorio-retina through the conjunctive route using two steps. The first step is the sequestration of the drug in the orbit because of vasoconstriction. The second step involves the transfer of the drug to the choroid because of oncotic pressure. Alpha adrenergic agents induce tension in the Bruck Wallace muscle and then in the bruck membrane. As a result choroid hydrostatic pressure decreases and oncotic pressure becomes relatively more important. Choroid arteries constriction by alpha adrenergic or beta-blocking agents increases arterial resistance, decreases hydrostatic pressure in capillaries and venules, so that oncotic pressure becomes relatively higher than the hydrostatic pressure, thus increasing the diffusion of the drug for treatment from the orbit to the chorio- retina and optic nerve head.

In the examples below, the following formulations were administered topically to patients:
- apraclonidine (lopidine®; alpha adrenergic agonist agent): 0.5% (w/v);
- bevacizumab (Avastin®; anti-VEGF agent): 2% (w/v);
- brimonidine (Alphagan®; alpha adrenergic agonist agent): 0.2% (w/v);
- dexamethasone (Tobradex®; corticosteroid): 0.1% (w/v);
- fluorescein: 10% (w/v);
- indomethacin (Indocollyre®; non-steroidal anti-inflammatory agent): 0.1% (w/v);
- neosynephrine (alpha adrenergic agonist agent): 10% (w/v);
- prednisolone (corticosteroid): 5.5% (w/v);
- ramipril (an angiotensin converting enzyme inhibitor): 2% (w/v);
- timolol (beta-blocking agent): 0.5% (w/v).

Unless otherwise indicated, administration of theses drugs was performed as follows:
- each patient received topically, in one eye, one drop of each of the indicated drug(s) (apraclonidine, bevacizumab, brimonidine, dexamethasone, indomethacin, neosynephrine, prednisolone, ramipril and/or timolol); then,
- every hour, over a period of 7 hours, the conjunctivae of the two eyes were exposed to one drop of fluorescein solution;
- 8 hours after administration of the first drug (apraclonidine, bevacizumab (apraclonidine, bevacizumab, brimonidine, dexamethasone, indomethacin, neosynephrine, prednisolone, ramipril and/or timolol), the fundus fluorescence in the two eyes was measured and analysed.

### Reference example 1

The experimental technique was based on ocular fundus fluorescence: Five patients were used in this study. Each patient received topically in one eye a drop of either apraclonidine or brimonidine or neosynephrine or timolol. Then every hour, the conjunctivae of the two eyes were exposed every hour to a 10% fluorescein solution. 8 hours later the fundus fluorescence in the two eyes was measured and analysed.

The fluorescence in the eye which received the drug carrier (brimonidine, apraclonidine, neosynephrine, or timolol) was stronger than in the eye receiving only fluorescein indicating that this drug carrier had enhanced the delivery of 10% fluorescein to the posterior segment (chorio-retina; optic nerve head). The following is a synopsis and results of the study undertaken:

### First case: lopidine and ocular fundus

| | |
|---|---|
| Right eye: | left eye: |
| lopidine +fluorescein | fluorescein |

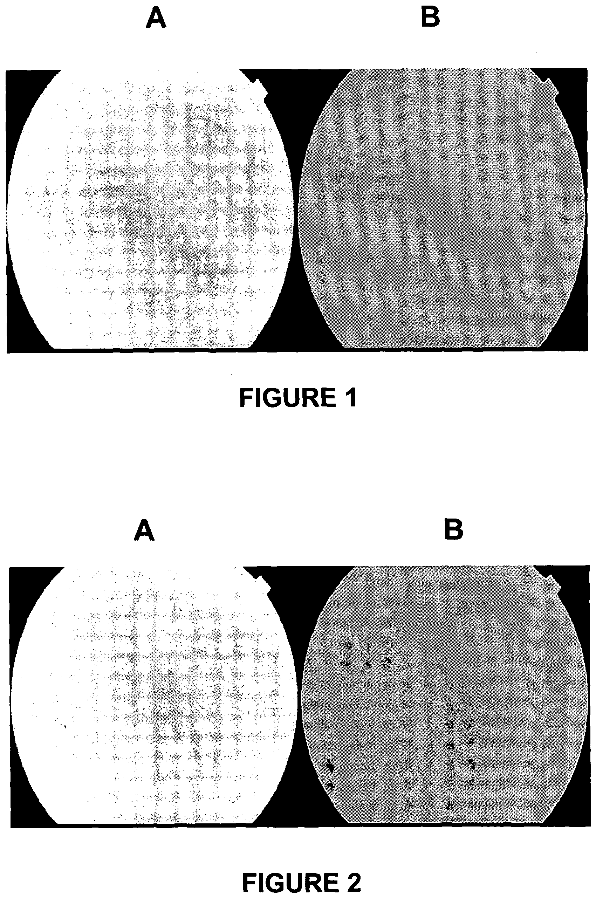

The results of the fundus fluorescence are shown in Fig.1. Fig. 1A is the result obtained using apraclonidine and fluorescein. Fig. 1B is the result obtained using only fluorescein. These results show that the fluorescence is stronger in the eye that was administered iopidine and fluorescein.

### Second case: Neosynephrine and ocular fundus

| | |
|---|---|
| Right eye: | left eye: |
| Neosynephrine + fluorescein | fluorescein |

The results of the fundus fluorescence are shown in Fig.2. Fig. 2A is the result obtained using neosynephrine and fluorescein. Fig. 2B is the result obtained using only fluorescein. These results show that the fluorescence is stronger in the eye that was administered neosynephrine and fluorescein.

### Third case: Timolol and ocular fundus and anterior segment

| | |
|---|---|
| Right eye: | left eye: |
| fluorescein | timolol + fluorescein |

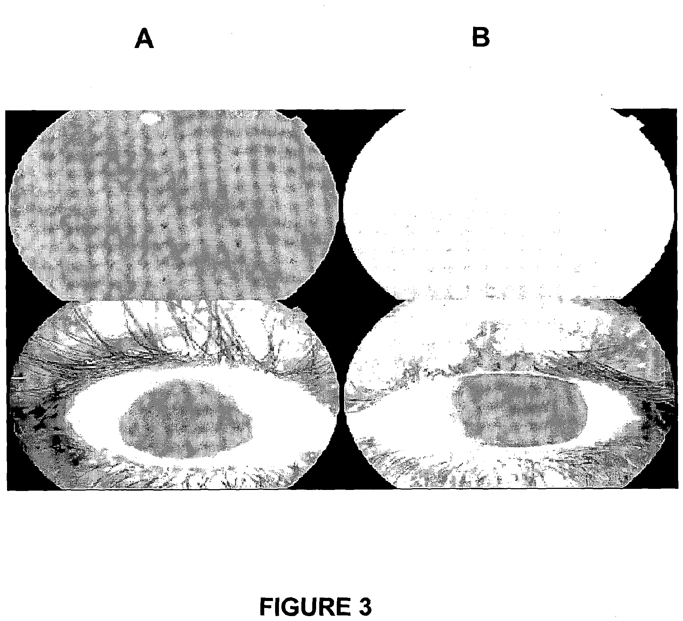

The results of the fundus fluorescence are shown in Fig.3. Fig. 3A is the result obtained using only fluorescein. Fig. 3B is the result obtained using only timolol and fluorescein. These results show that the fluorescence is stronger in the eye that was administered timolol and fluorescein (ocular fundus). However, fluorescence is the same in both anterior segments.

### Forth case: Apraclonidine and ocular fundus and anterior segment

| | |
|---|---|
| Right eye: | left eye: |
| fluorescein | Apraclonidine + fluorescein |

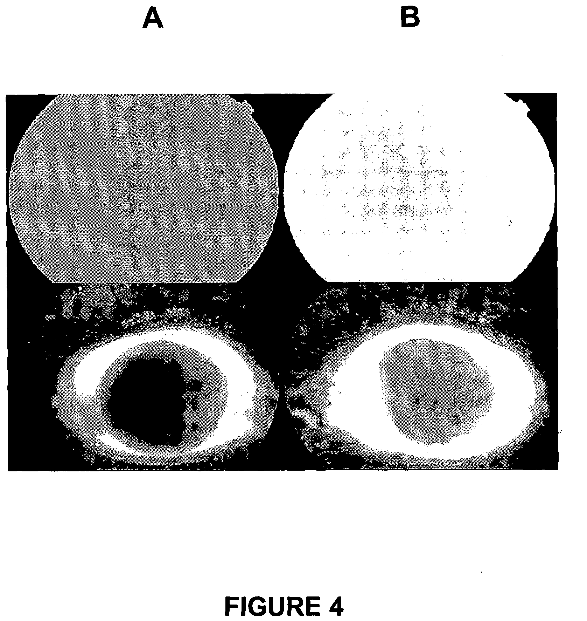

The results of the fundus fluorescence are shown in Fig.4. Fig. 4A is the result obtained using only fluorescein. Fig. 4B is the result obtained using apraclonidine and fluorescein. These results show that the fluorescence is stronger in the eye that received apraclonidine and fluorescein (ocular fundus and anterior segment).

### Fifth case: Brimonidine and ocular fundus and anterior segment

| | |
|---|---|
| Right eye: | left eye: |
| fluorescein | Brimonidine + fluorescein |

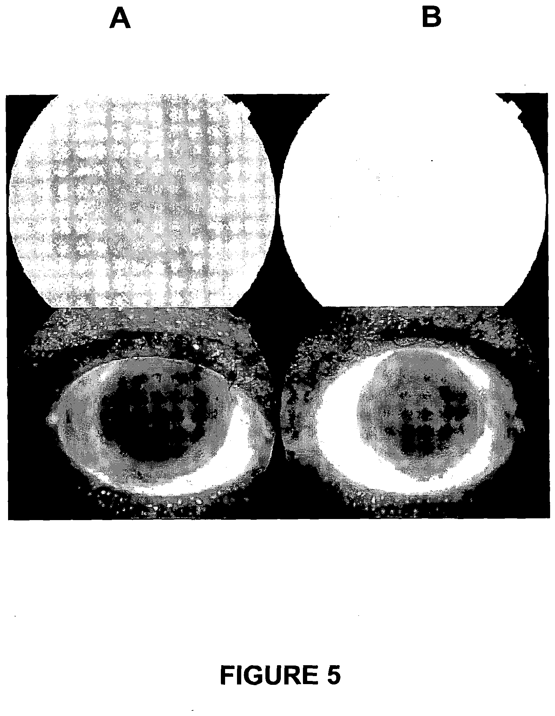

The results of the fundus fluorescence are shown in Fig.5. Fig. 5A is the result obtained using only fluorescein. Fig. 5B is the result obtained using brimonidine and fluorescein. These results show that the fluorescence is stronger in the eye that was administered brimonidine and fluorescein (ocular fundus and anterior segment).

Collectively, these results demonstrated that the fluorescence is stronger in the eye which received the drug carrier. It works out that exposure of the conjunctival fornices to a 10% fluorescein solution leads to a maximum vitreous concentration of 1.5 x 10⁻¹² g/ml, 7 hours later. For fluorescein this brings it into the range of therapeutic concentrations.

### Reference example 2

### DIABETIC RETINOPATHY

Diabetic retinopathy is the leading cause of new blindness in individuals under 65 years of age. Diabetic retinopathy can be classified into non-proliferative diabetic retinopathy (NPDR) and proliferative diabetic retinopathy (PDR). The clinical features of NPDR include microaneurysms, intraretinal hemorrhages, hard exudates, nerve fiber layer infarcts or cotton wool exudates and intra retinal microvascular abnormalities (IRMA). The clinical picture of PDR includes the features from NPDR in addition to proliferating new vessels on the optic nerve head, retina or iris.

Diabetic macular oedema is a principal cause of visual loss in diabetic patients. Two examination techniques are very useful in evaluating diabetic retinopathy: fluorescein angiography and optical coherence tomography.

Fluorescein angiography is used to detect several of the retinal vascular abnormalities. The dye delineates structural vascular alterations, such as aneurysms or neovascularization, changes in blood flow such as ischemia and vascular occlusion are seen as an interruption of the normal perfusion pattern. Abnormal vascular permeability is seen as a leaking cloud of dye-stained oedema fluid increasing overtime.

Optical coherence tomography (O.C.T) may be more sensitive in evaluating diabetic macular oedema than slit- lamp examination. In addition, central macular thickness correlates with visual acuity even better than fluorescein leakage.

The response of macular oedema to the administration of one drug such as an anti-VEGF agent or an anti-inflammatory treatment such as topical corticosteroids, non-steroidal anti-inflammatory agents or angiotensin converting enzyme inhibitors, can be documented accurately by OCT imaging.

Diabetic patients were treated with one of the following drugs given topically: a corticosteroid (dexamethasone (Tobradex®) or prednisolone), a non steroidal anti-inflammatory agent (indomethacin (Indocollyre ®)), an anti-VEGF (bevacizumab (Avastin®)) or an angiotensin converting enzyme inhibitor (Ramipril). These drugs were given in combination with a drug carrier: an alpha adrenergic agonist agent or a beta-blocking agent or alpha adrenergic agonist agent combined with a beta-blocker, for enhancing the delivery of the drug to the retina.

The following study was undertaken on six patients having non-proliferative diabetic retinopathy or proliferative diabetic retinopathy.

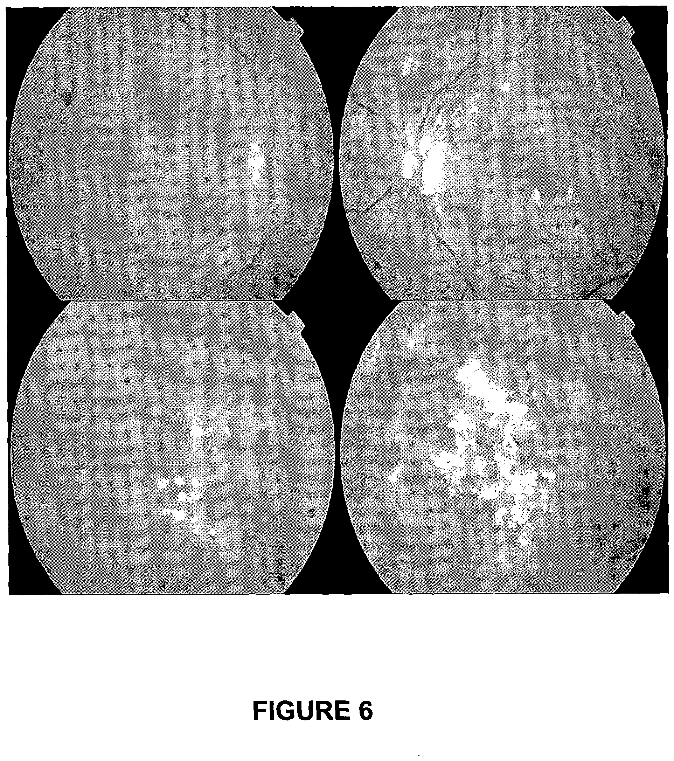

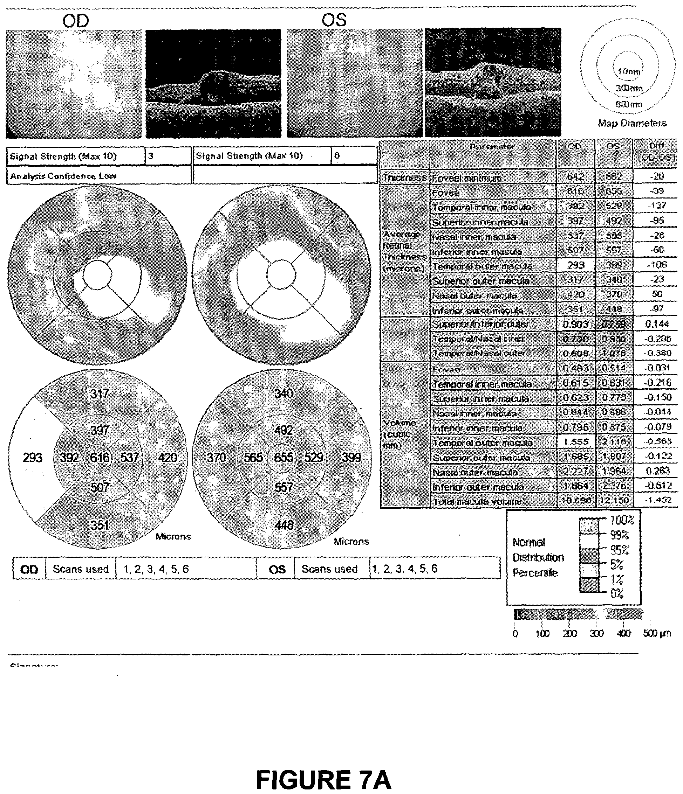

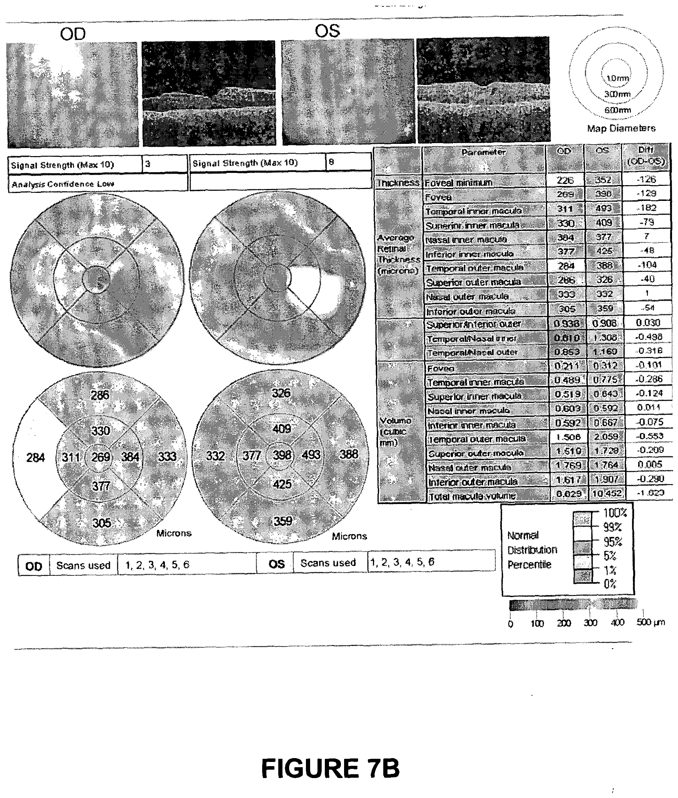

B.C.N is a diabetic patient who presented with a diabetic retinopathy (Fig. 6: occular fundus and fluoro-angiography; fig. 7A: OCT). He received topically a combination of apraclonidine + bevacizumab (Avastin®). Two months after treatment, a follow up OCT scan (Fig. 7B) showed that the total macula volume had decreased to 285 microns in the right eye and 402 microns in the left eye explaining visual improvement.

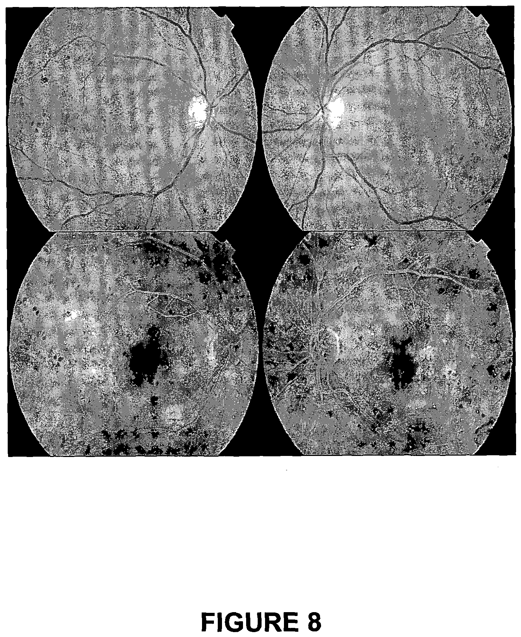

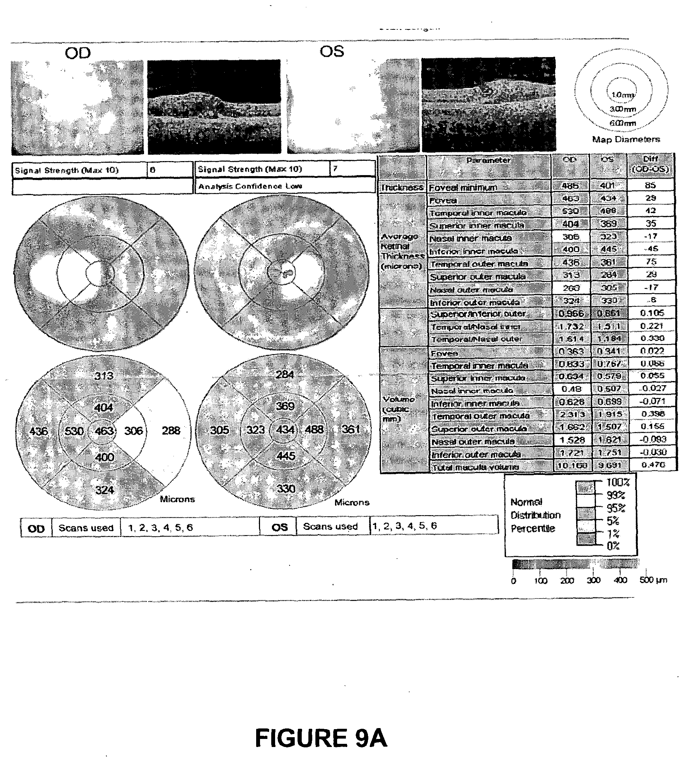

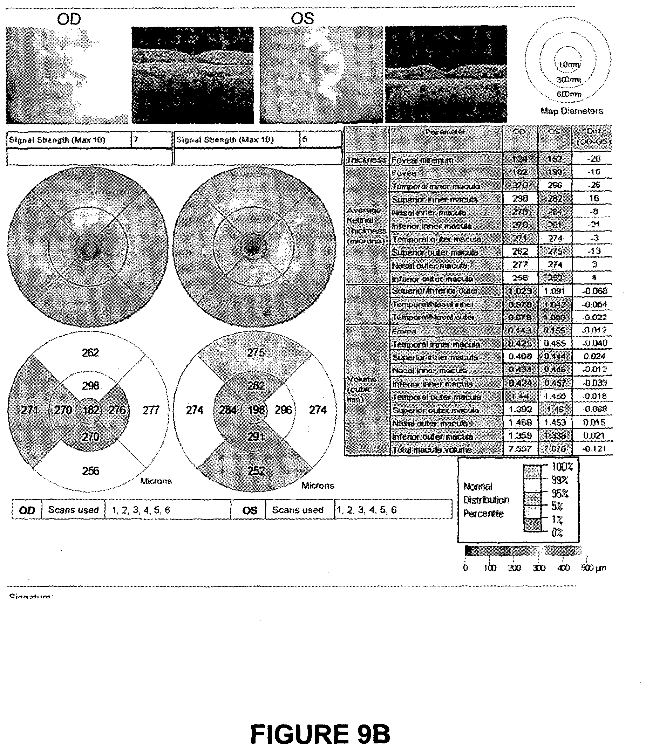

Patient G.N presented with a non proliferative diabetic retinopathy (figure 8: occular fundus and fluoro-angiography; figure 9A: OCT). She received topically: corticosteroid + non-steroidal anti-inflammatory agent + apraclonidine. Three months later, her visual acuity was improved. A follow up OCT scan three months later (Fig. 9B) showed complete resolution of the intra- retinal and subretina fluid.

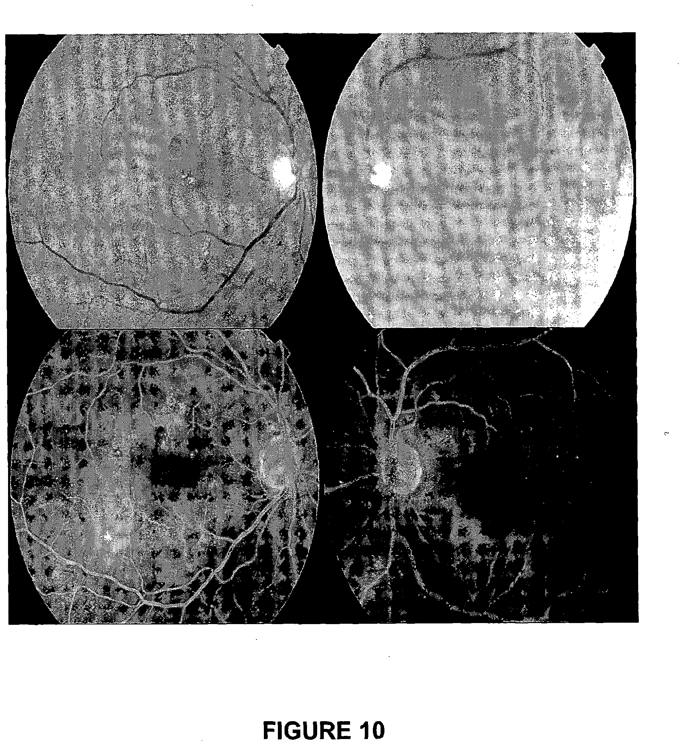

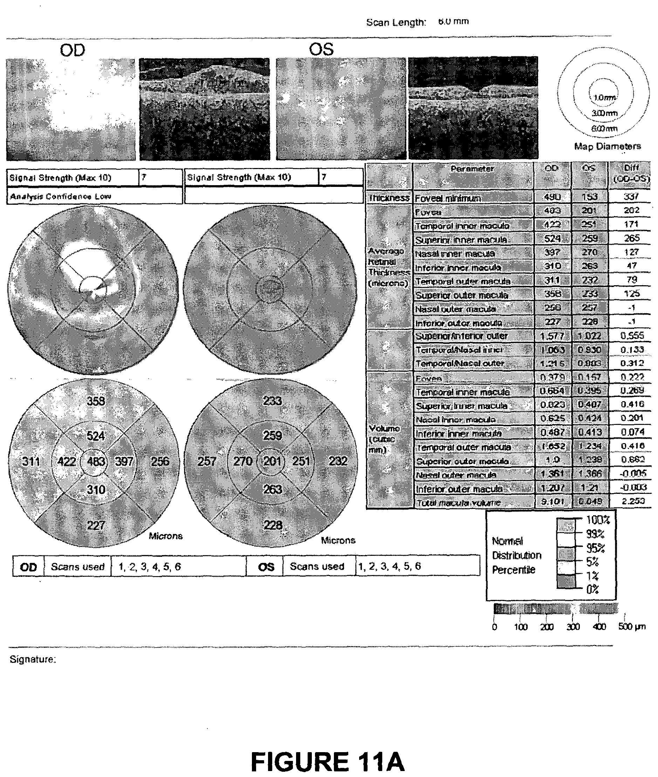

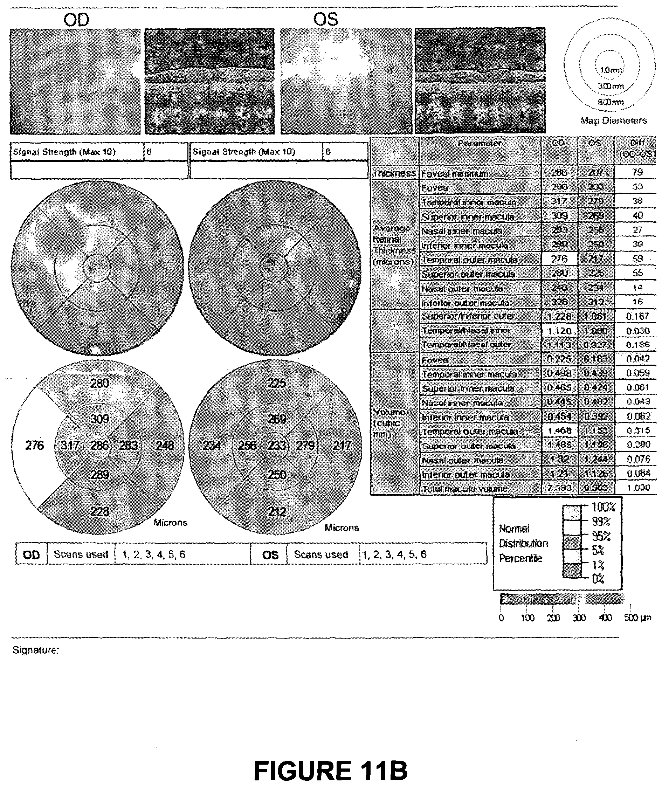

Patient M.M. presented with a non proliferative diabetic retinopathy (Fig. 10: ocular fundus and fluoro-angiography; Fig. 11A: OCT). She received topically: corticosteroid + neosynephrine. Three months later, her visual acuity improved. A follow up OCT scan (Fig. 11B) showed an almost complete resolution of the intraretinal fluid.

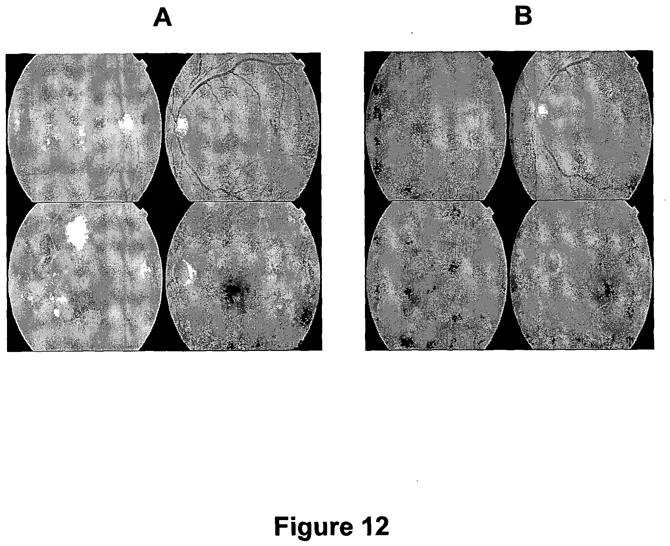

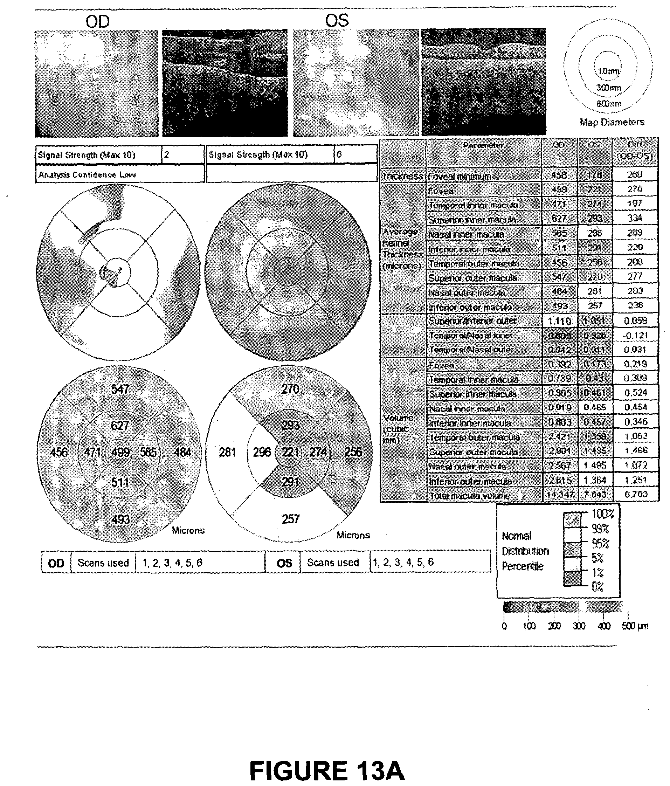

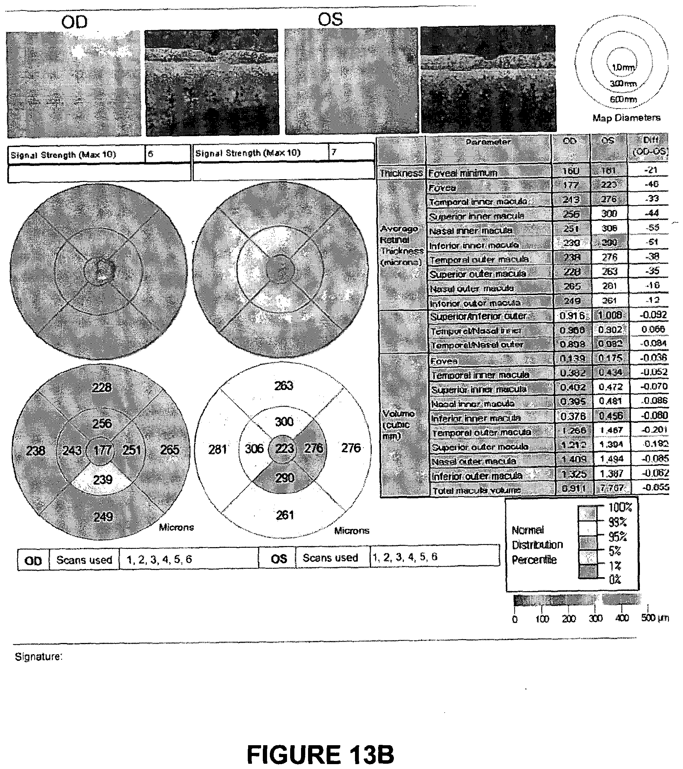

Patient H.M. presented with a proliferative diabetic retinopathy. He received a combination of brimonidine + a non-steroidal anti-inflammatory agent. An ocular fundus and fluoro-angiography were performed prior to treatment and the results are shown in Fig. 12A. Prior to the treatment and three months after the treatment, an OCT scan was taken, which is shown in Fig. 13A and 13B respectively. Visual acuity improved after treatment. A follow up fluoro-angiography (Fig. 12B) showed the regression of new vessels and macular oedema: the retinal map analysis revealed foveal normalization.

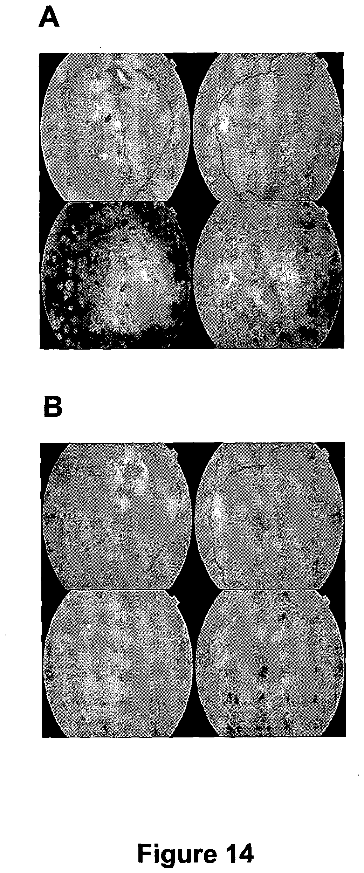

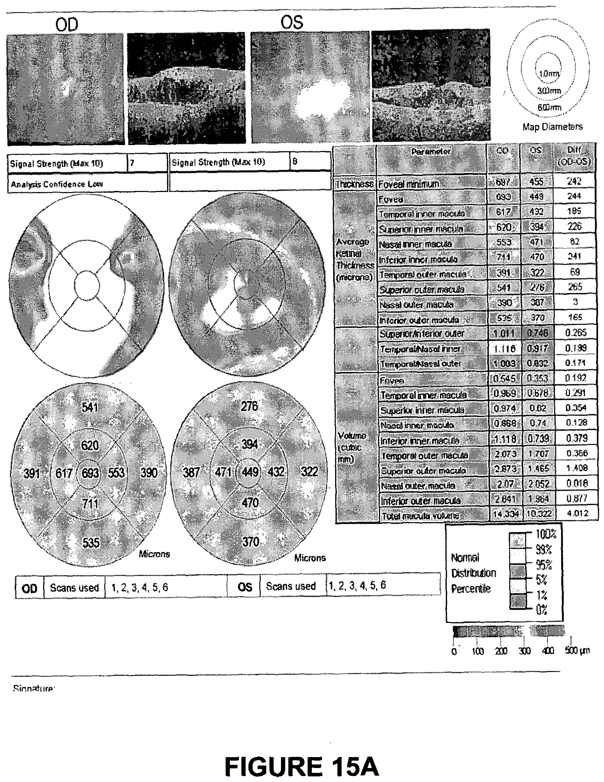

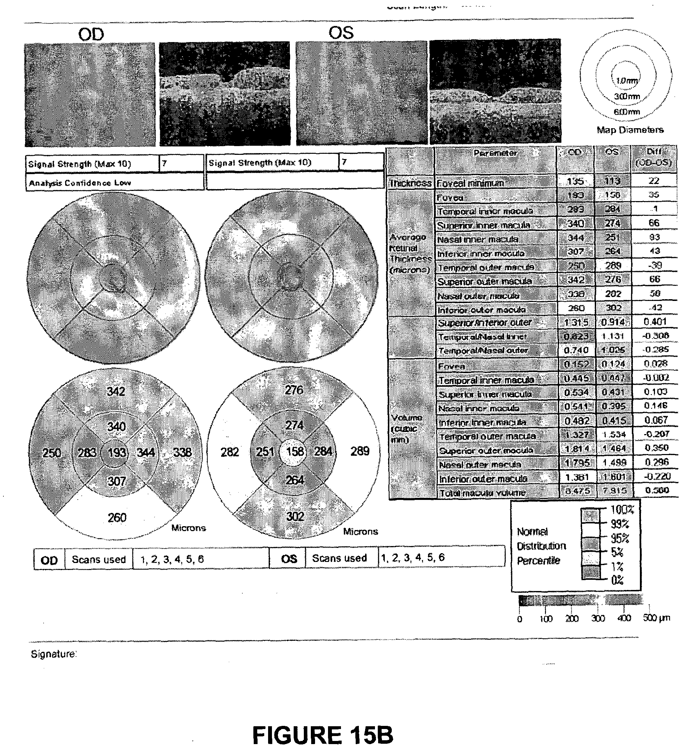

Patient N.B presented with a non proliferative diabetic retinopathy. She received topically: corticosteroid + non-steroidal anti-inflammatory agent + brimonidine. An ocular fundus and fluoro-angiography were performed prior to treatment and the results are shown in Fig. 14A. Prior to receiving the treatment an OCT scan was also taken, which is shown in Fig. 15A. Six months later her visual acuity improved. A follow up fluoro-angiography (Fig. 14B) and OCT (Fig. 15B) demonstrated a reduction of macular oedema and foveal thickening.

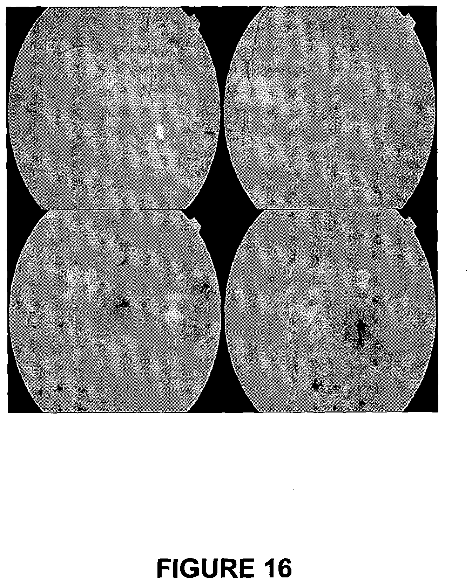

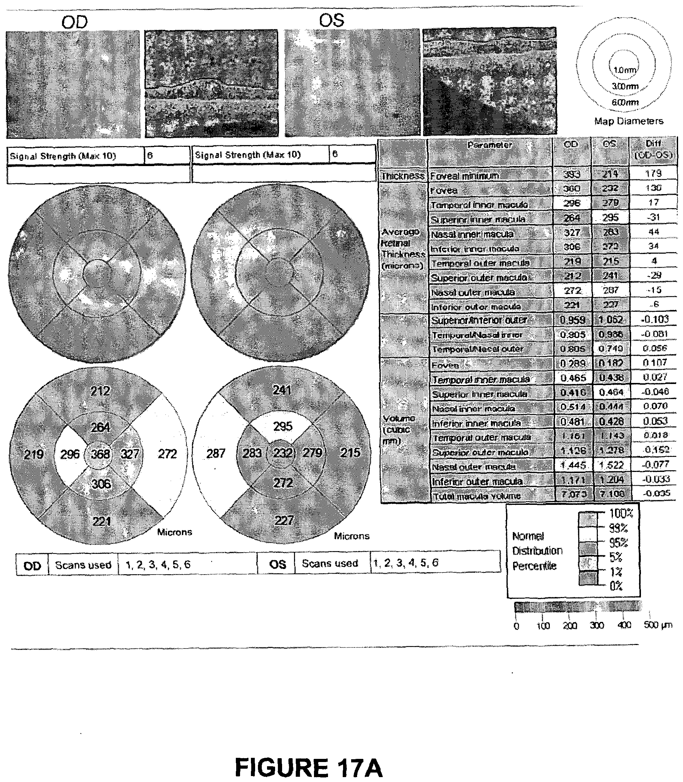

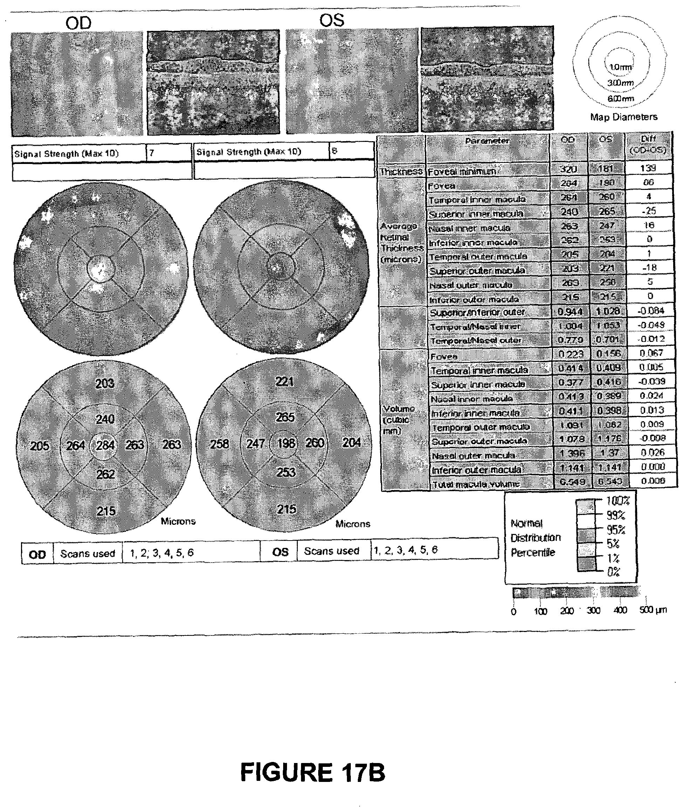

Patient M.R. presented with a diabetic retinopathy. He received topically: apraclonidine + Avastin® (anti-VGEF). Prior to receiving the treatment an OCT scan was taken, which is shown in Fig. 17A. An ocular fundus and fluoro-angiography were performed prior to treatment and the results are shown in Fig. 16. Three months later, his visual acuity improved. A follow up OCT (Fig. 17B) demonstrated and a reduction of macular oedema and foveal thickening.

As shown from the results above, the patients improved their vision with a decrease of macular oedema and neovascularization after 2 months of treatment. A follow-up OCT scan showed that the foveal thickness had decreased. An almost complete resolution of macular oedema had also been observed.

### Example 3

### RETINAL VEIN OCCLUSION

Central retinal vein occlusion (CRVO) is a common retinal vascular condition usually affecting people older then 50 years. Patients typically experience visual loss and present with dilated tortuous retinal veins and scattered intra-retinal hemorrhage in all four quadrants, cotton wool spots, optic disc swelling, and macular oedema can occur. Intra veinous fluorescein angiography shows areas of blocked fluorescence from the intra-retinal blood, staining of the vessel walls, a delayed arteriovenous phase, and nonperfused areas, and perifoveal leakage.

OCT detects macular oedema. Recent studies have shown the efficacy of intravitreal triancinolone (Aristocert®) injection in macular oedema secondary to CRVO. An anti-VEGF agent (Avastin®) when injected into the eye improved this condition.

12 patients, instead of injection, received topically either a corticosteroid (dexamethasone (Tobradex®) or prednisolone) or an anti-VEGF agent (Avastin®), associated with a delivery drug enhancer such as an alpha adrenergic agonist agent alone or combined with a beta-blocking agent.

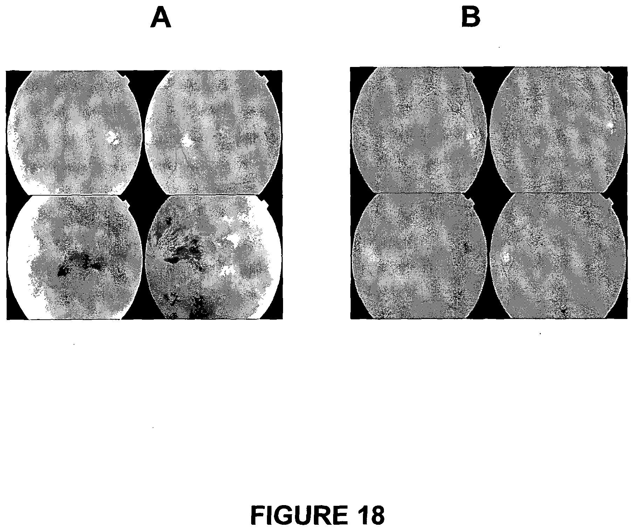

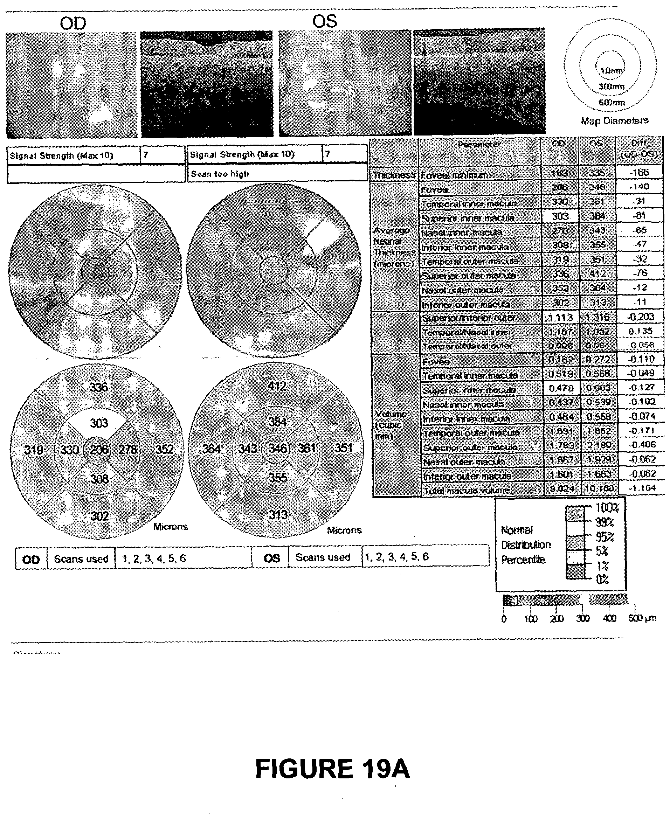

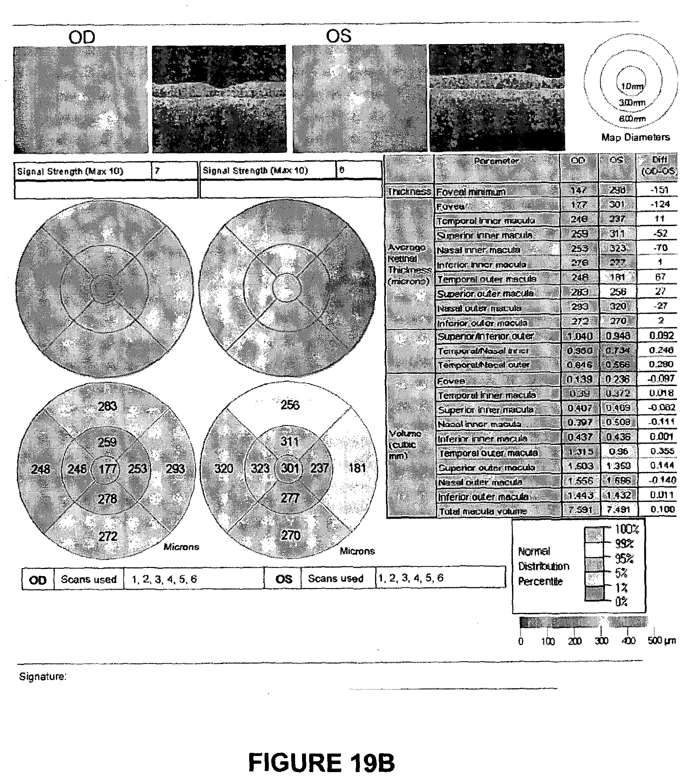

Patient N.B. presented with non proliferative diabetic retinopathy; branch vein occlusion in the left eye. She received topically: corticosteroid + non-steroidal anti-inflammatory agent + neosynephrine. Prior to receiving the treatment, an OCT scan was taken, which is shown in Fig.19A. An ocular fundus and fluoro-angiography were performed prior to treatment and the results are shown in Fig. 18A. Three months later her visual acuity improved. A follow up fluoro-angiography (Fig. 18B) and OCT (Fig. 19B) demonstrated a reduction of macular oedema and foveal thickening.

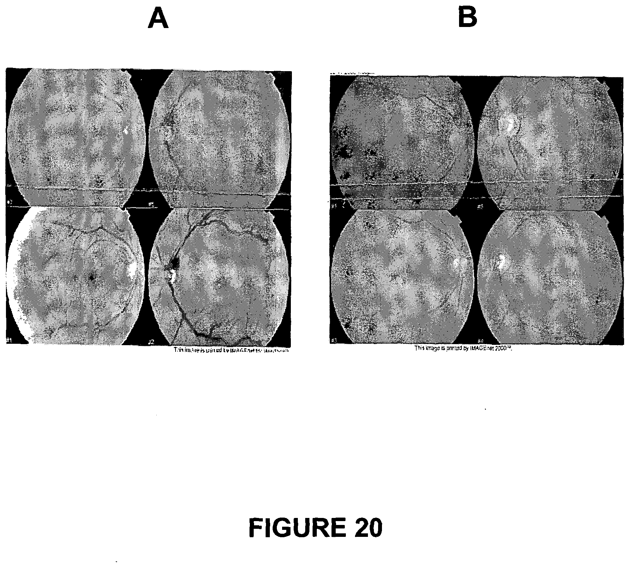

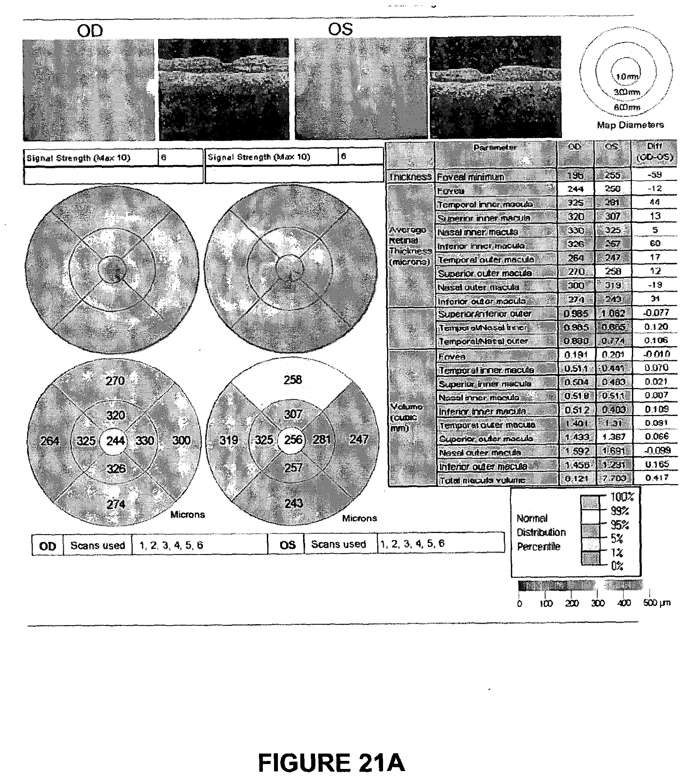

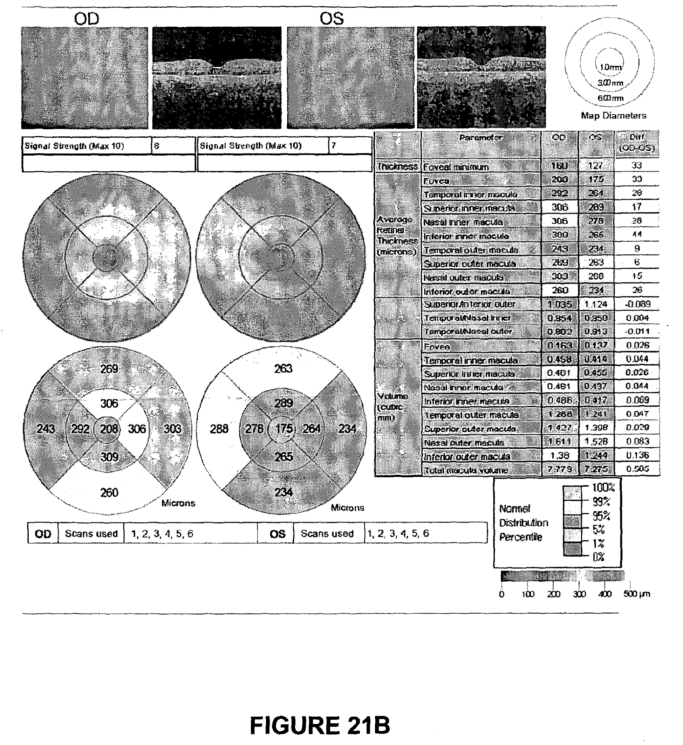

Patient B.S.T presented with imminent central retinal vein occlusion in the left eye. He received topically a non-steroidal anti-inflammatory agent + apraclonidine. Prior to receiving the treatment an OCT scan was taken, which is shown in Fig. 21A. An ocular fundus and fluoro-angiography were performed prior to treatment and the results are shown in Fig. 20A. Two weeks later his visual acuity improved. A follow up ocular fundus exam (Fig. 20B) and OCT (Fig. 21B) demonstrated the normalization of the left eye.

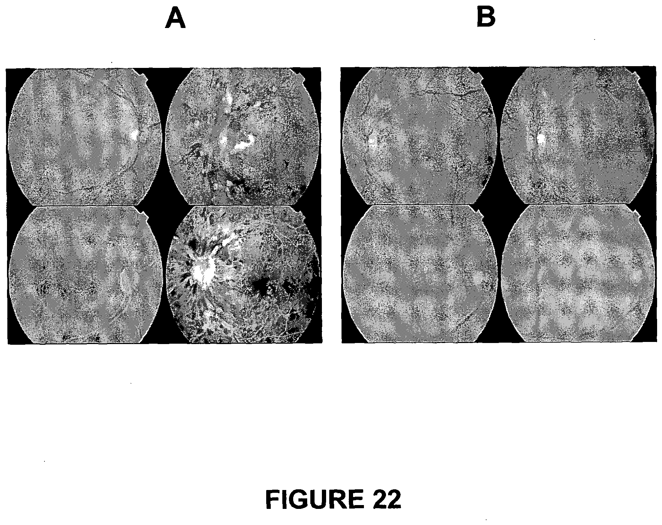

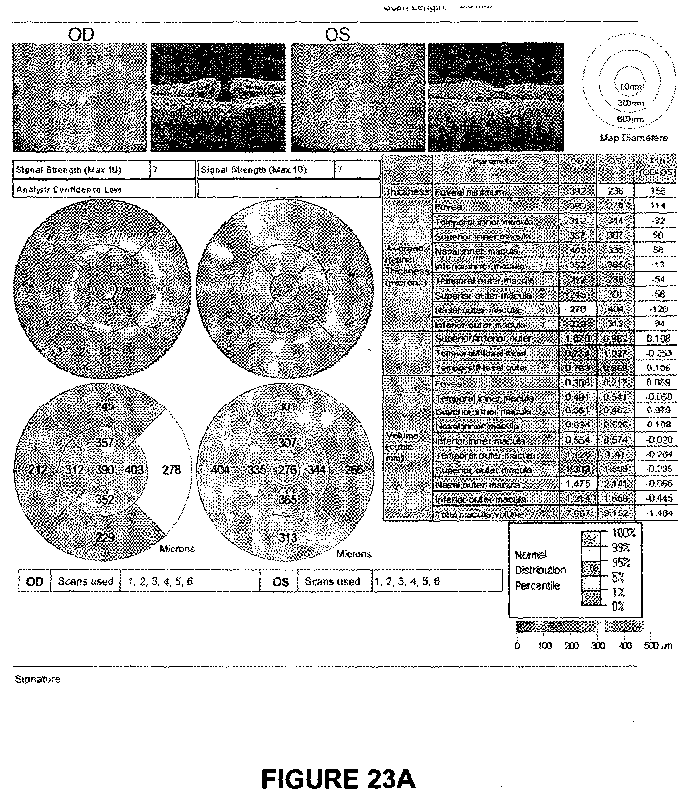

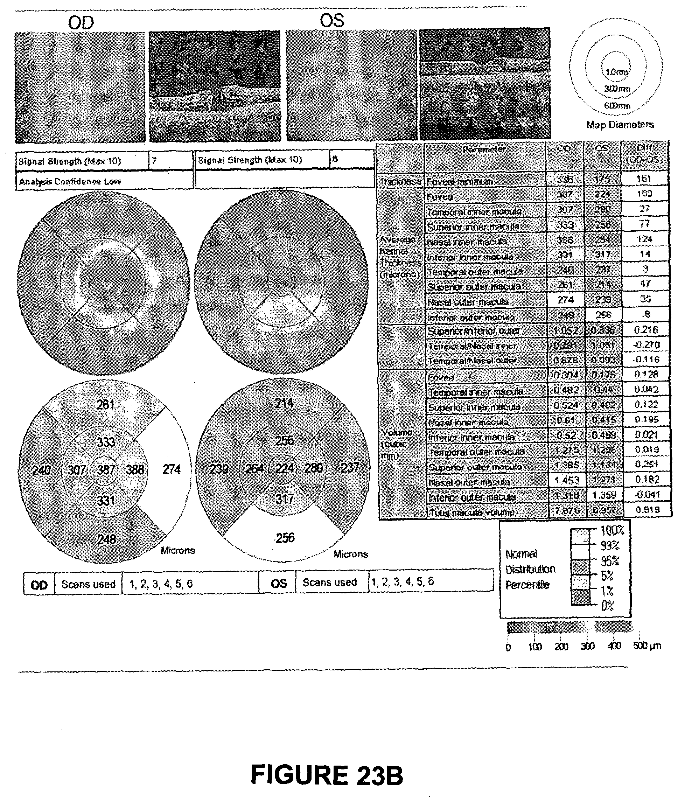

Patient A.H presented with a central retinal vein occlusion in the left eye. He received topically: non-steroidal anti-inflammatory agent + corticosteroid + neosynephrine. Prior to receiving the treatment an OCT scan was taken, which is shown in Fig. 23A. An ocular fundus and fluoro-angiography were performed prior to treatment and the results are shown in Fig. 22A. Three months later, his visual acuity increased. A follow up ocular fundus exam (Fig. 22B) revealed a fundus normalization and OCT scan (Fig. 23B) demonstrated reduction in foveal thickening.

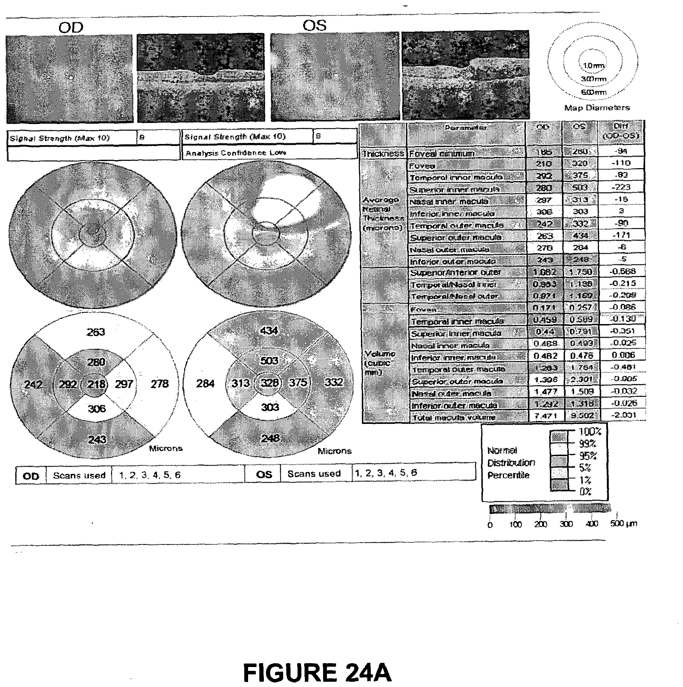

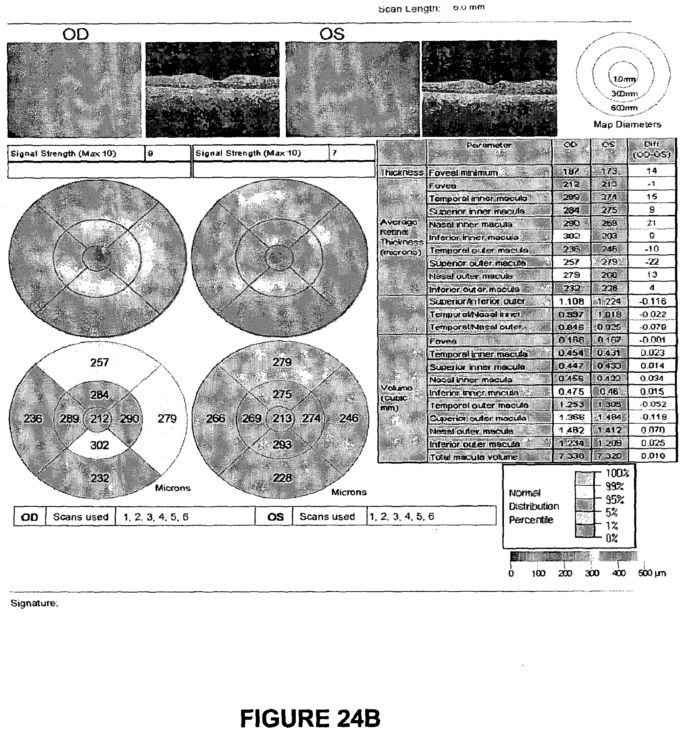

Patient S.Z presented with a branch retinal occlusion in the left eye. He received topically: Angiotensin converting enzyme inhibitor (ramipril) + apraclonidine. Prior to receiving the treatment, an OCT scan was taken, which is shown in Fig. 24A. Three months later his visual acuity increased. A follow up OCT scan (Fig. 24B) demonstrated regression in macular thickening.

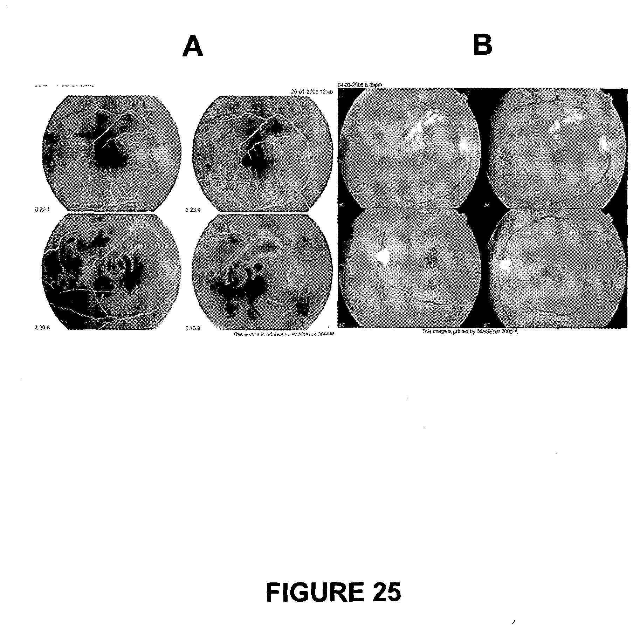

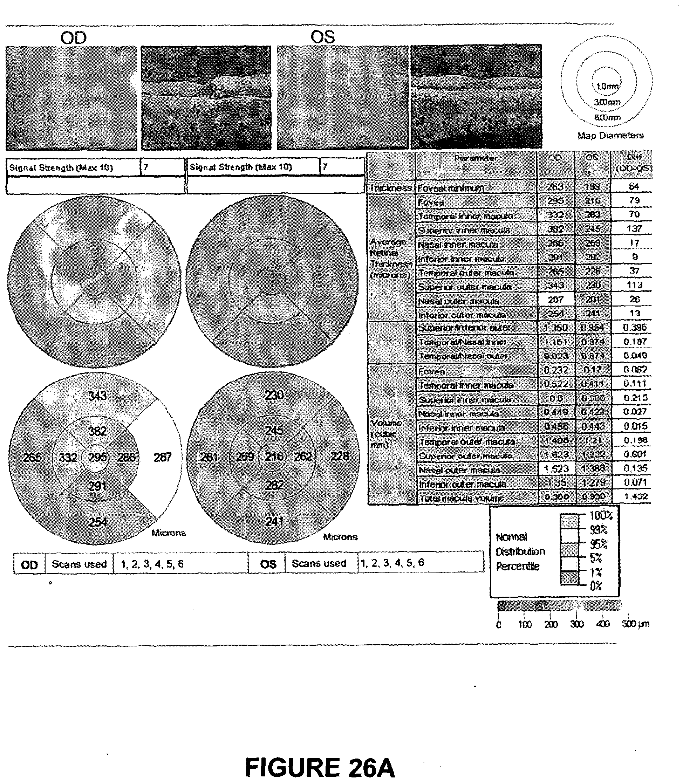

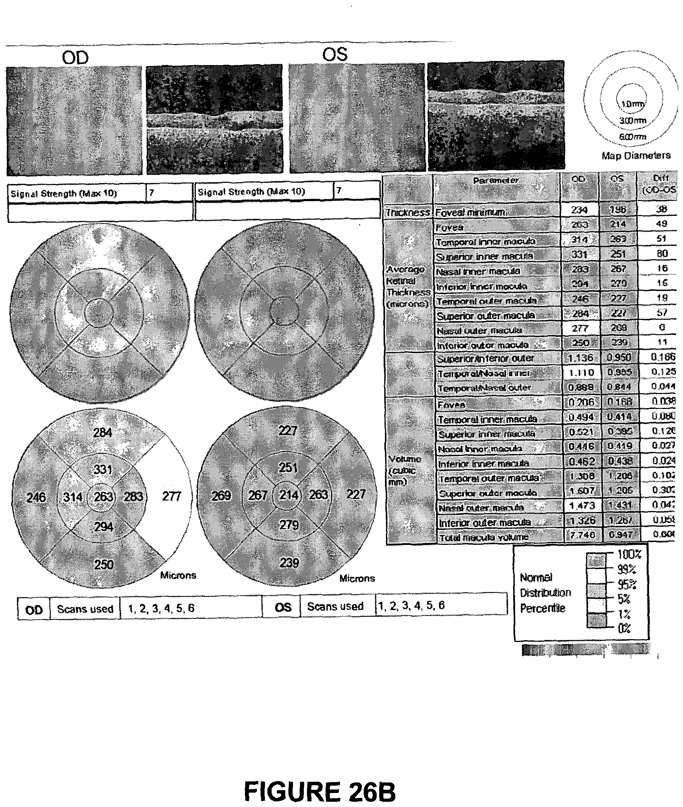

Patient D.M.H presented with a branch retinal occlusion in the right eye. He received topically: Avastin® (anti-VEGF) + neosynephrine. Prior to receiving the treatment an OCT scan was taken, which is shown in Fig. 26A. An ocular fundus and fluoro-angiography were performed prior to treatment and the results are shown in Fig. 25. One month later his visual acuity increased. A follow up OCT exam revealed an improvement of the macular oedema (Fig. 26B).

As seen from the above results the patients improved their condition of visual acuity, angiographic feature and OCT results when administered the regular drug and the carrier drug.

### Reference example 4

### AGE - RELATED MACULAR DEGENERATION (ARMD)

Age related macular degeneration (ARMD) is the leading cause of severe vision loss among the elderly. The cause of ARMD remains elusive and complex, with both environmental and genetic contributions. ARMD has two distinct forms known as "dry" or non-neovascular ARMD and "wet", or neovascular ARMD. Most the severe vision loss in ARMD is caused by neovascular ARMD.

The best proven therapies for ARMD treat the neovascular form of the disease and include photocoagulation triamcinolone intraocular injection and anti-VEGF intraocular injection (Avastin®; Lucentis®; Macugen®).

The structural information provided by OCT is becoming a valuable diagnostic adjunct to fluorescein angiography. OCT is a valuable tool for probing the effects of these treatments.

Instead of an intraocular injection of an anti-VEGF agent, a corticosteroid, a non-steroidal anti-inflammatory agent and/or an angiotensin converting enzyme inhibitor, 10 patients received topically either a corticosteroid (dexamethasone (Tobradex®) or prednisolone), an anti-VEGF (bevacizumab (Avastin®)), a non steroidal anti-inflammatory agent (indomethacin (Indocollyre ®)) and/or an angiotensin converting enzyme inhibitor (Ramipril), associated with a delivery drug enhancer such as an alpha adrenergic agonist agent alone or in combination with a beta-blocking agent.

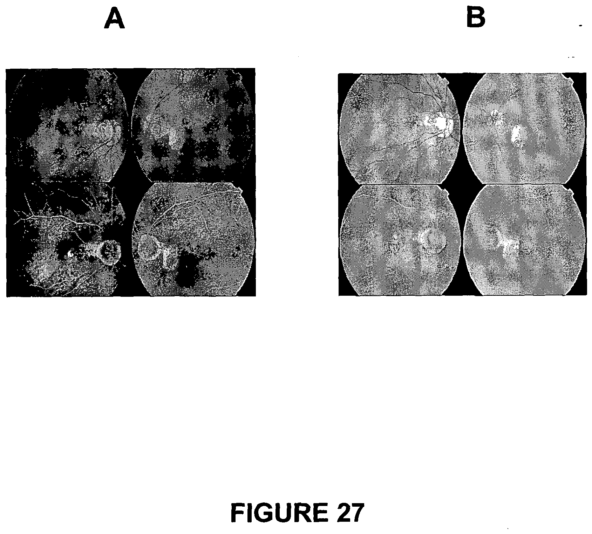

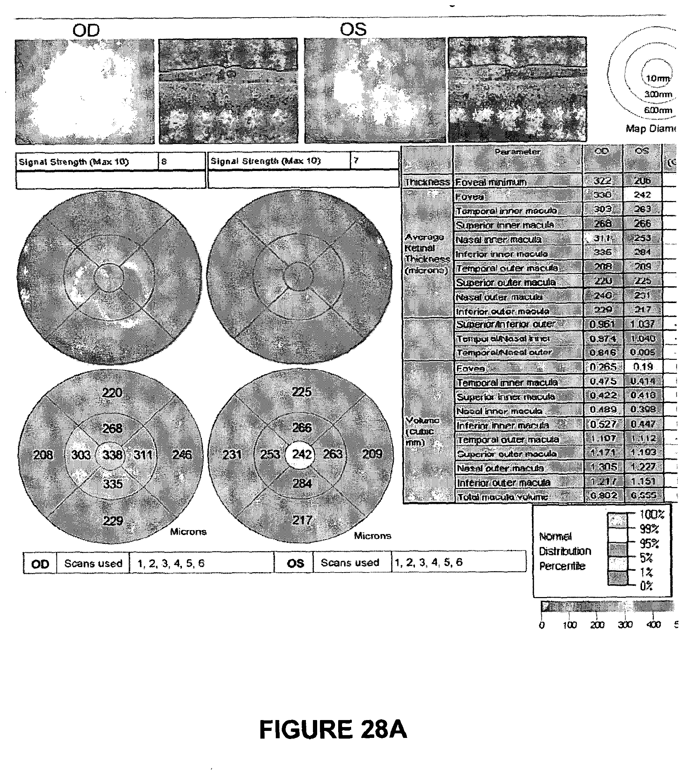

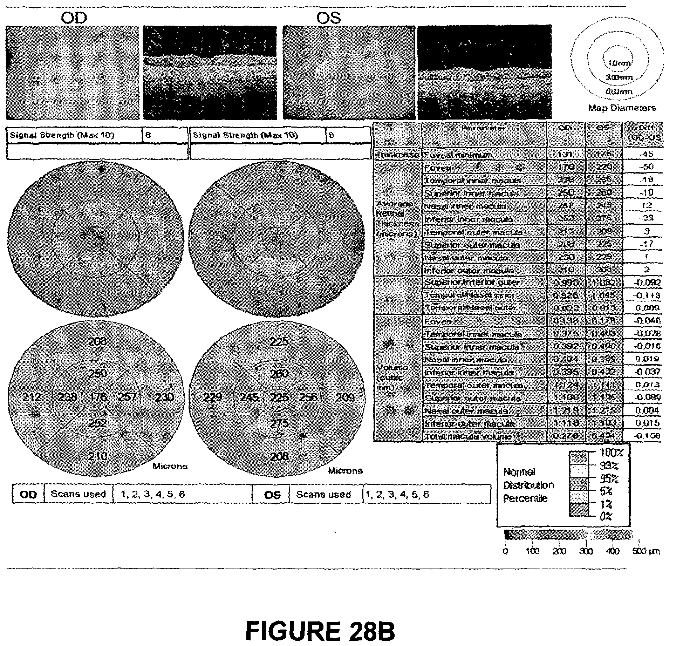

Patient CH.A presented with a two-month history of decreased vision in the right eye. An early angiographic image showed well delineated lacy subfoveal choroid new vessel (CNV) (Fig. 27A). An OCT exam showed retinal thickening with a loss of foveal contour (fig. 28A). The patient was treated with corticosteroid + non-steroidal anti-inflammatory agent + apraclonidine. Two months after treatment, the patient's vision improved. Fluoro-angiography revealed a decreased activity of the new vessel (Fig. 27B). Follow up OCT scan (Fig. 28B) demonstrated a complete regression of macular thickening.

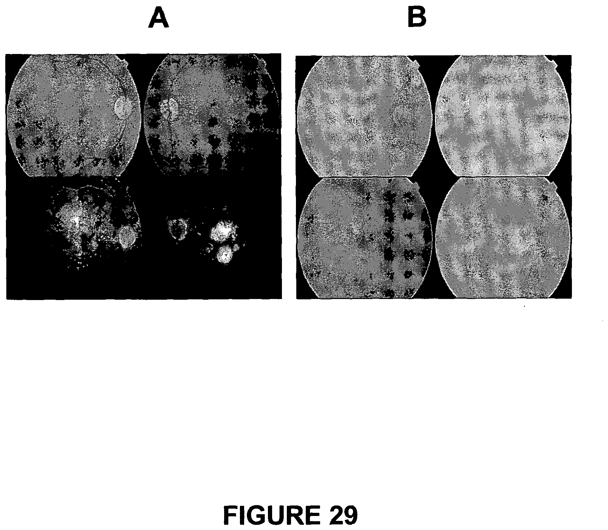

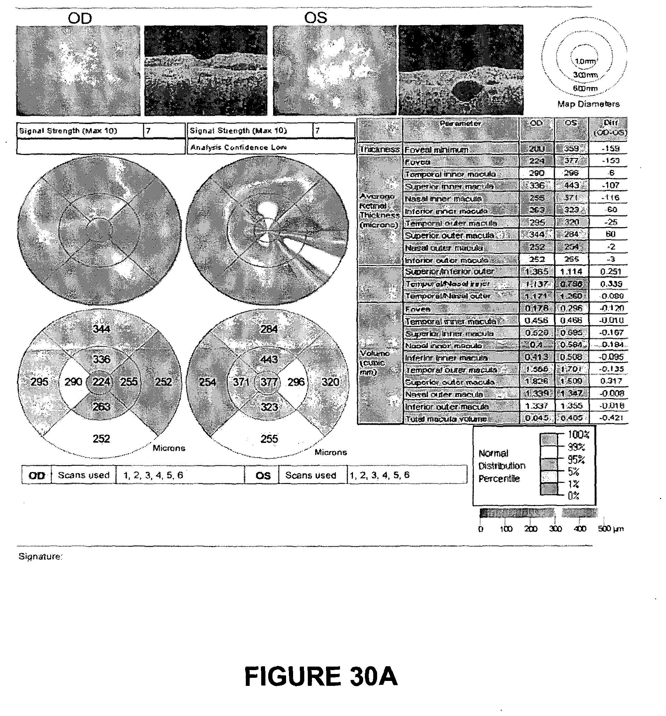

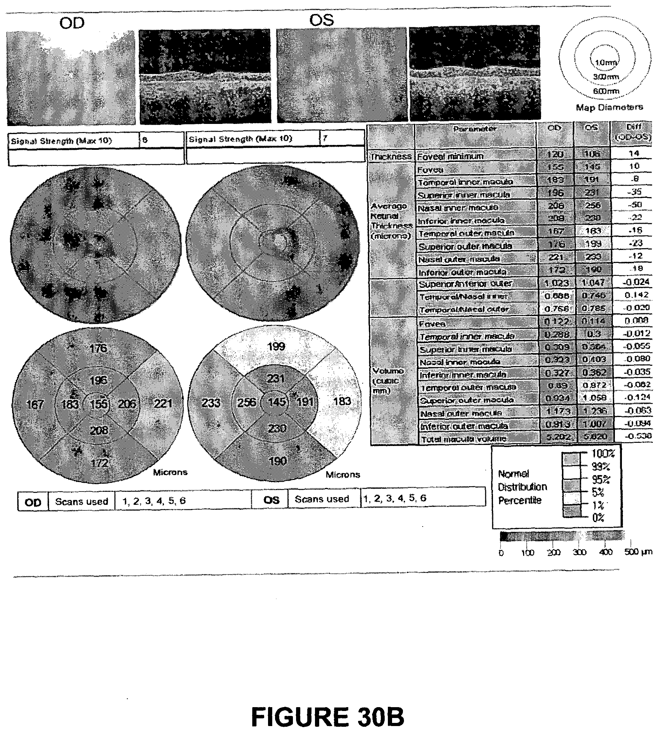

Patient K.T presented with a three-month history of decreased vision in both eyes. A fluoro-angiographic image showed choroid occult new vessel (CNV) with pigmentary epithelium detachment (Fig. 29A). An OTC exam revealed a macular thickening and a pigmentary epithelium detachment (Fig. 30A). He was treated with corticosteroid + non-steroidal anti-inflammatory agent + neosynephrine. Three months after treatment, the patient's vision improved. Fluoro-angiography revealed a decreased activity of the new vessel (Fig. 29B). A follow up OCT scan (Fig. 30B) demonstrated a complete regression of macular thickening and recovery of foveal contour.

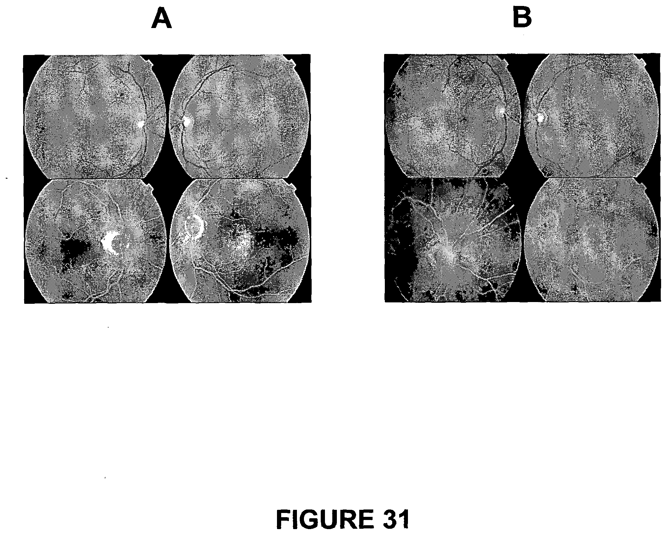

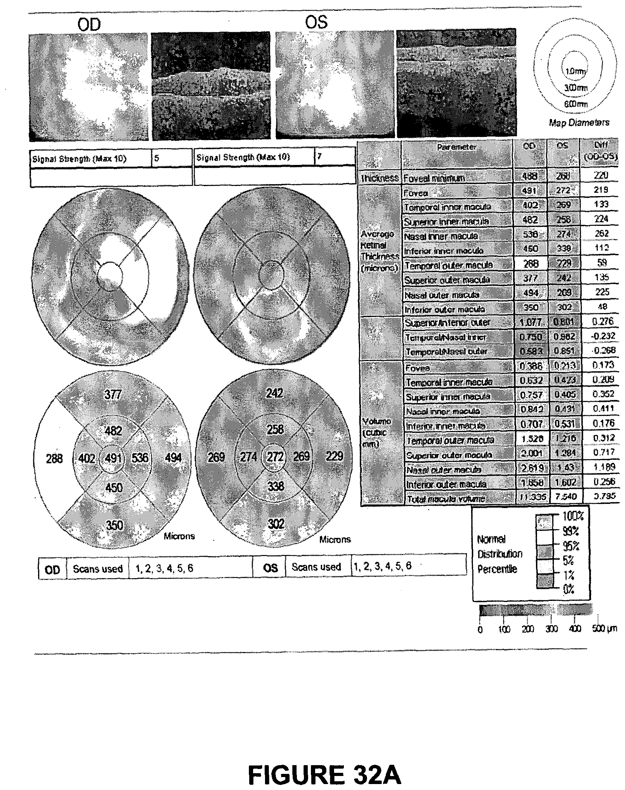

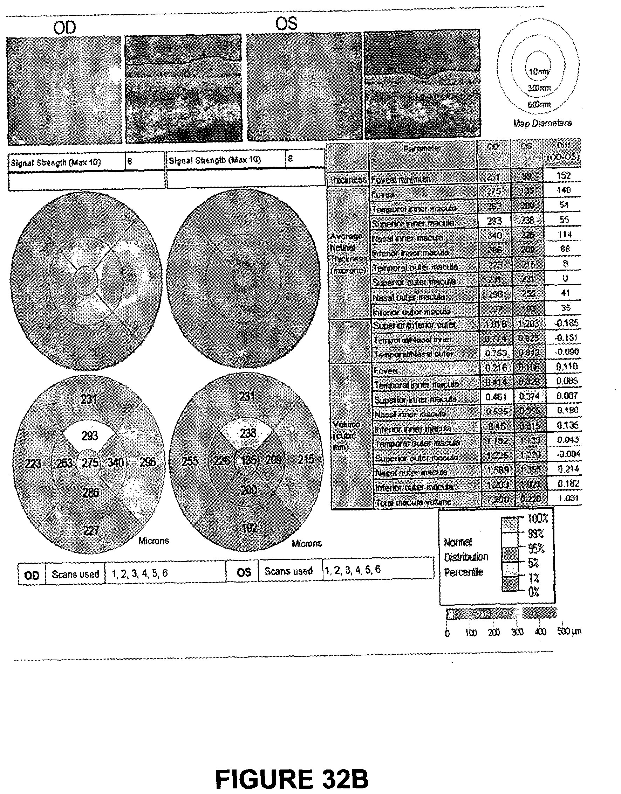

Patient D.F presented with a history of decreased vision in the left eye. A fluoro-angiographic image showed choroid occult new vessel (CNV) (Fig. 31A). Prior to receiving the treatment an OCT scan was taken, which is shown in Fig. 32A. She was treated with corticosteroid + non-steroidal anti-inflammatory agent + apraclonidine. Three months after treatment, the patient's vision improved. Fluoro-angiography revealed a decreased activity of the new vessel (Fig. 31B). Follow up OCT (Fig. 32B) demonstrated a complete regression of macular thickening and recovery of foveal contour.

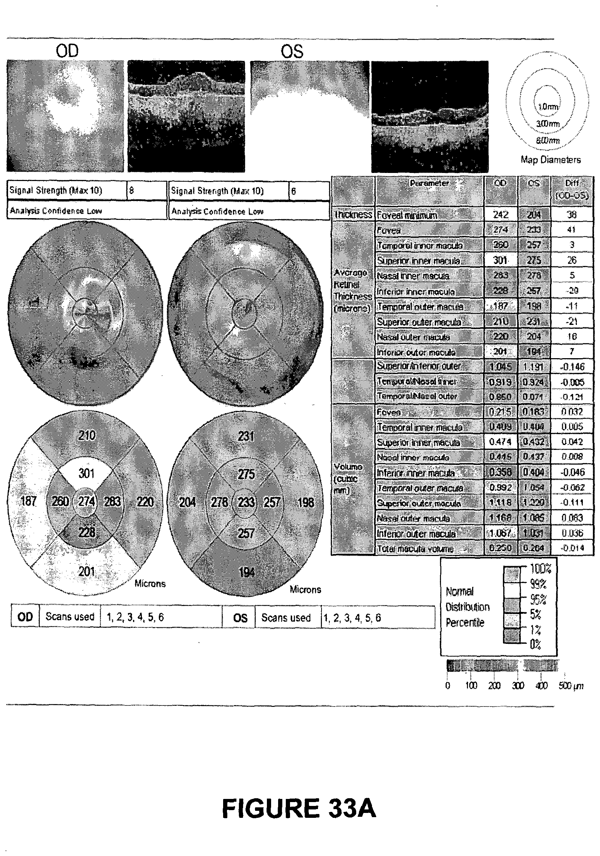

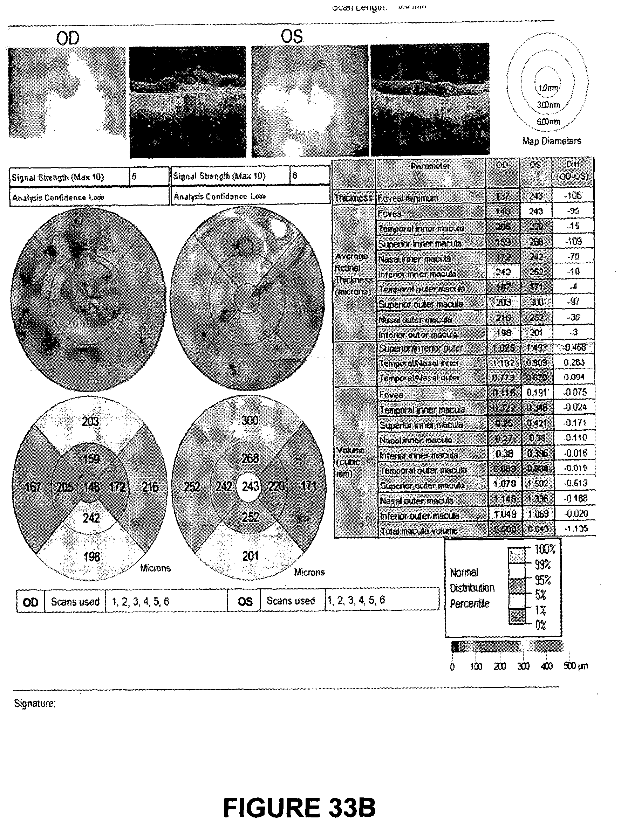

Patient D.M presented with a history of decreased vision in the right eye. A fluoro-angiographic image showed choroid occult new vessel (CNV) (image not shown). Prior to receiving the treatment an OCT scan was taken, which is shown in Fig. 33A. He was treated with Avastin® (anti-VGEF) + apraclonidine. One month after treatment, the patient's vision improved. Fluoro-angiography revealed a decreased activity of the new vessel (image not shown). Follow up OCT (Fig. 33B) demonstrated a complete regression of macular thickening and recovery of foveal contour.

As can be ascertained from the above data the patients improved their vision. Fluoresceinangiography and OCT showed a decrease of choroidal new vessels and macular thickening.

### Example 5

### MISCELLANEOUS MACULAR DEGENERATIONS

In addition to age-related macular degeneration (ARMD), numerous conditions have been associated with the development of chroidal neovascularization (CNV). These conditions, which primarily affect younger patients, include pathologic myopia.

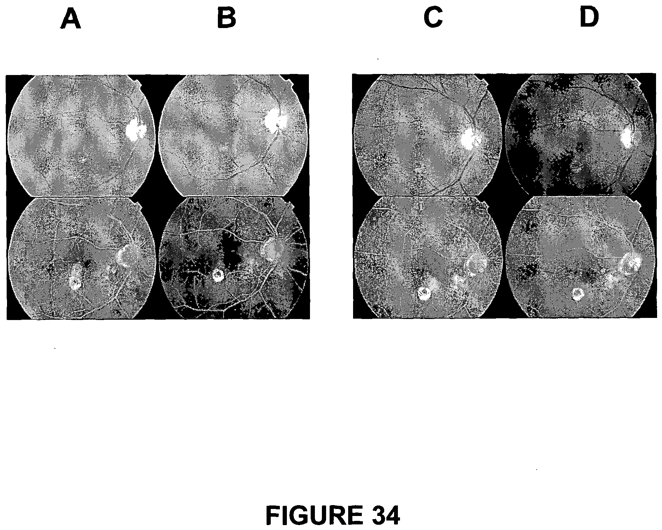

Patient B.S.S. is a thirty year old male who had complaints of decreased central vision in the right eye. Fluorescence-angiography showed early hyperfluorescence with late leakage consistent with subfoveal classic CNV (choroid new vessel) (Fig. 34A). He was treated topically with Avastin® (anti-VGEF) + apraclonidine. Two months later (Fig. 34B), visual acuity improved and new vessel activity regressed. Four months later and because he stopped the treatment (Fig. 34C) a decreased visual acuity associated with recurrence of new vessel activity was observed. He received topically Avastin® (anti- VGEF) + apraclonidine. Two weeks later (Fig. 34D) visual acuity improved and new vessel activity stopped.

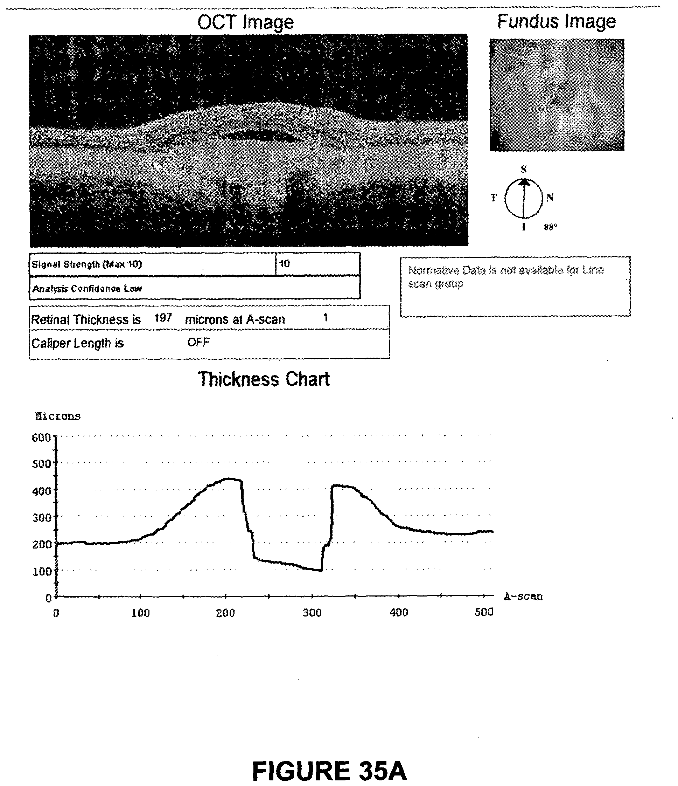

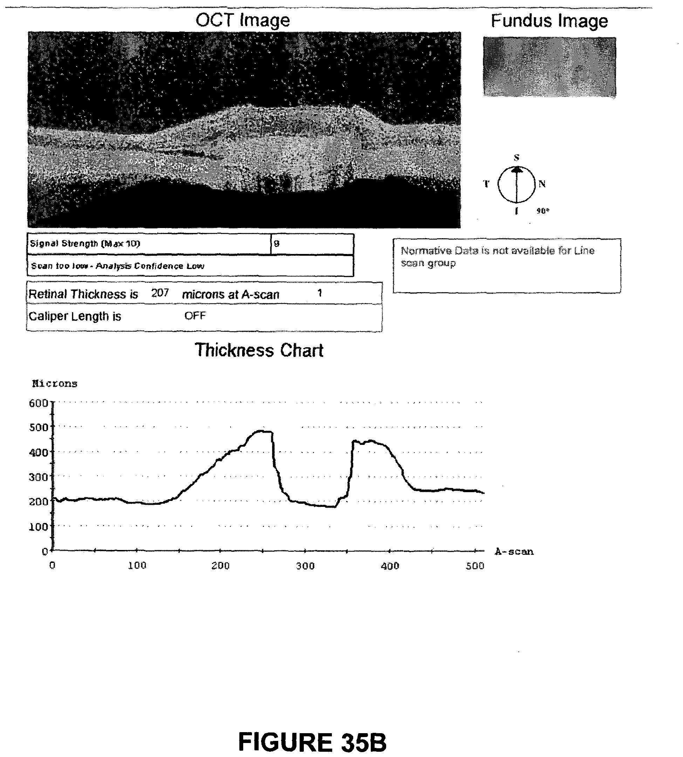

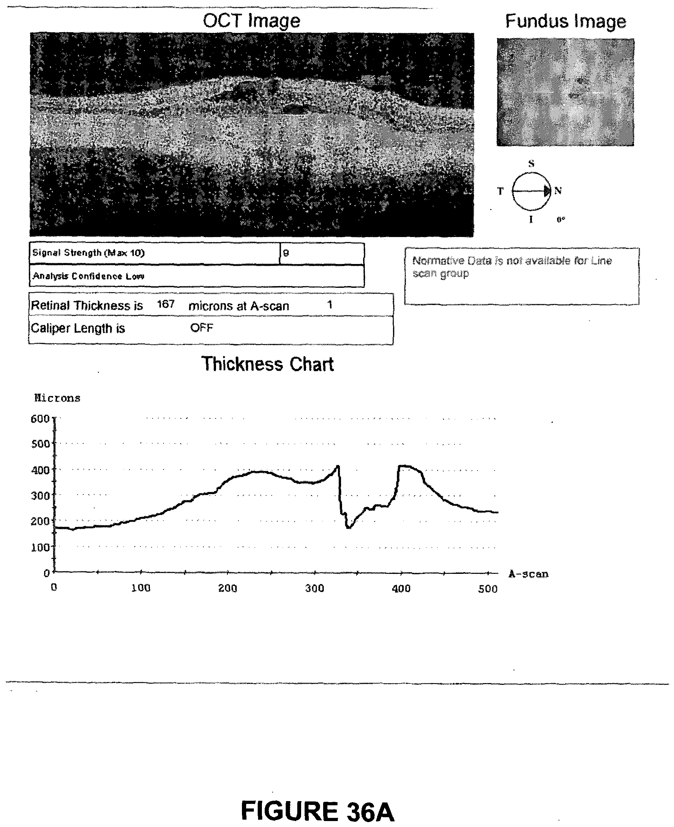

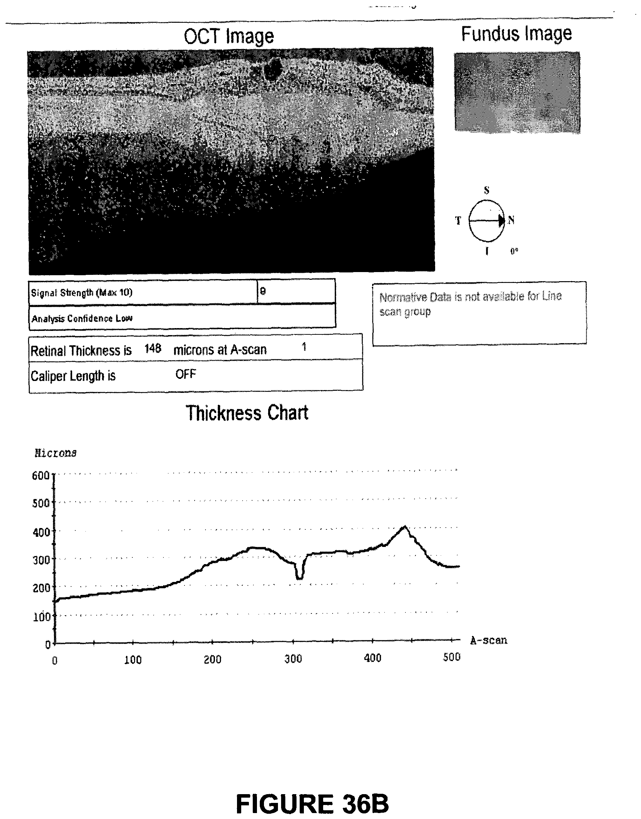

Patient G.C is a twenty nine year old female who had complaints of decreased central vision in the right eye because of classic macular new vessels. The OCT tomogram (Figs. 35A and 36A) showed an oval hypereflective lesion. There was a pocket of subretinal fluid adjacent to this lesion and mild overlying retinal edema. She was treated with ramipril (Angiotensin converting enzyme inhibitor) + timolol + neosynephrine. One week later visual acuity improved and resolution of the retinal edema at the OCT control was noted (Figs. 35B and 36B).

### Example 6

### GLAUCOMA NEUROPATHY

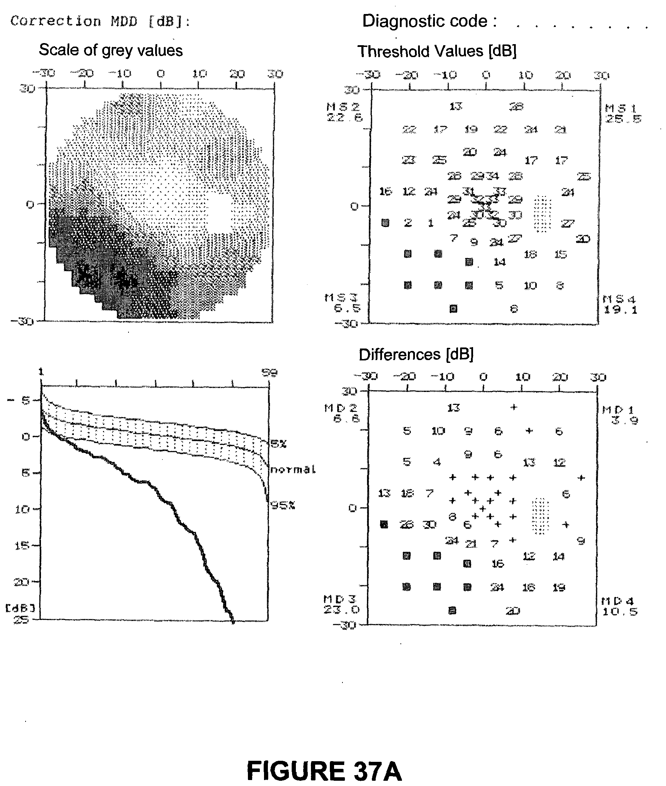

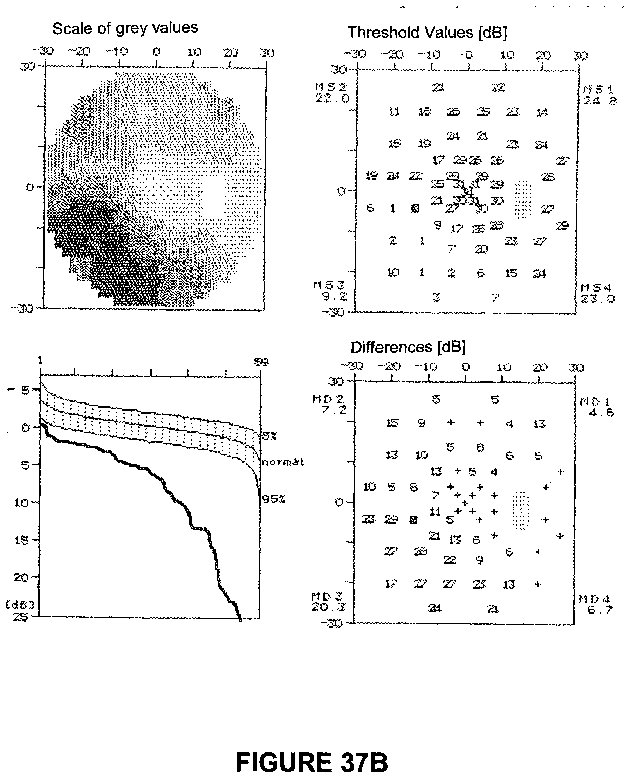

Patient A.L presented with an open angle chronic glaucoma in the right eye. Despite surgical normalization of her intraocular pressure she continued to deteriorate in her field of vision (Fig.37A). She received topically ramipril (Angiotensin converting enzyme inhibitor) + brimonidine. Two weeks later we noted an improvement of nasal scotoma (Fig. 37B).

In summary of these results showed that the use of a carrier drug such as an alpha adrenergic agonist agent and/or a beta-blocking agent alone or in combination resulted in effective delivery of drugs to treat eye disorders and/or eye diseases.

## Claims

1. A kit comprising or consisting of:
a) ramipril and apraclonidine, or
b) ramipril and brimonidine, or
c) ramipril, neosynephrine, and timolol,
in which apraclonidine, brimonidine, neosynephrine, and timolol can be administered either simultaneously or prior to ramipril.

2. The kit according to claim 1, wherein apraclonidine, brimonidine or neosynephrine are dosed at physiologically acceptable amounts of 0.01% to 20% (w/v), preferably from 0.1% to 15% (w/v), and more preferably from 0.1 to 3% (w/v), timolol is dosed at a physiologically acceptable amount of 0.05% to 2% (w/v), preferably 0.1 % to 1 % (w/v), and more preferably from 0.1% to 0.5% (w/v), and ramipril is dosed at a concentration ranging from 0.001 to 15% (w/v), preferably from 0.05 to 10% (w/v), and more preferably from 0.1 to 3% (w/v).

3. The kit according to any one of claim 1 or 2, further comprising an ophtalmologically acceptable carrier, such as water, especially distilled or deionized water, a saline solution, especially a phosphate buffered saline solution, or excipient(s).

4. The kit according to any one of claims 1 to 3, comprising or consisting of ramipril dosed at 2% (w/v) and apraclonidine dosed at 0.5% (w/v).

5. The kit according to any one of claims 1 to 3, comprising or consisting of ramipril dosed at 2% (w/v) and brimonidine dosed at 0.2% (w/v).

6. The kit according to any one of claims 1 to 3, comprising or consisting of ramipril dosed at 2% (w/v), neosynephrine dosed at 10% (w/v), and timolol dosed at 0.5% (w/v).

7. An ophtalmologic composition comprising or consisting of the ingredients of a kit as defined in any one of claims 1 to 6.

8. The kit or ophthalmologic composition according to any one of claims 1 to 7, for use as a medicament.

9. The kit or ophthalmologic composition for use according to claim 8, for treating disorders and/or diseases of the eye.

10. The kit or ophthalmologic composition for use according to any one of claims 8 or 9, for topical administration.

11. The kit or ophthalmologic composition for use according to any one of claim 8 to 10, wherein apraclonidine, brimonidine, neosynephrine, and/or timolol contact(s) the eye prior to an effective amount of ramipril.

12. The kit or ophthalmologic composition for use according to any one of claims 8 to 11, for delivering ramipril to the posterior segment of the eyes, and in particular to the chorio-retina and optic nerve head of an eye.

13. The kit or ophthalmologic composition for use according to any one of claims 8 to 12, for the treatment of at least one eye disorder and/or disease of the eye selected from the group of glaucoma, glaucomatous neuropathy, diabetic retinopathy, retinal vein occlusion, in particular central retinal vein occlusion or branch retinal vein occlusion, macular oedema, dry and exsudative macular degeneration, in particular age related macular degeneration.

14. The kit or ophthalmologic composition for use according to any one of claims 8 to 13 wherein apraclonidine, brimonidine or neosynephrine is(are) administered to a person or an animal in need thereof at a concentration ranging from 0.01% to 20% (w/v), preferably from 0.1% to 15% (w/v), and more preferably from 0.1 to 3% (w/v), timolol is administered to a person or an animal in need thereof at a concentration ranging from 0.05% to 2% (w/v), preferably 0.1 % to 1 % (w/v), and more preferably from 0.1% to 0.5% (w/v), ramipril is administered to a person or an animal in need thereof at a concentration ranging from 0.001 to 15% (w/v), preferably from 0.05 to 10% (w/v), and more preferably from 0.1 to 3% (w/v).

## Patentansprüche

1. Kit, umfassend oder bestehend aus:
a) Ramipril und Apraclonidin oder
b) Ramipril und Brimonidin oder
c) Ramipril, Neosynephrin und Timolol,
in dem Apracloinidin, Brimonidin, Neosynephrin und Timolol entweder gleichzeitig oder vor Ramipril verabreicht werden können.

2. Kit gemäß Anspruch 1, in dem Apraclonidin, Brimonidin oder Neosynephrin in physiologisch akzeptablen Mengen von 0,01 % bis 20 % (Gew./Vol.), bevorzugt von 0,1 % bis 15 % (Gew./Vol) und weiter bevorzugt von 0,1 bis 3 % (Gew./Vol.) dosiert sind, Timolol in einer physiologisch akzeptablen Menge von 0,05 % bis 2 % (Gew./Vol.), bevorzugt von 0,1 % bis 1 % (Gew./Vol.) und weiter bevorzugt von 0,1 % bis 0,5 % (Gew./Vol) dosiert ist und Ramipril in einer Konzentration dosiert ist, die von 0,001 bis 15 % (Gew./Vol.), bevorzugt von 0,05 bis 10 % (Gew./Vol.) und weiter bevorzugt von 0,1 bis 3 % (Gew./Vol.) reicht.

3. Kit gemäß irgendeinem der Ansprüche 1 oder 2, weiterhin umfassend einen ophthalmologisch akzeptablen Träger, wie z. B. Wasser, insbesondere destilliertes oder entionisiertes Wasser, eine Salzlösung, insbesondere eine gepufferte Phosphat-Salzlösung oder (ein) Hilfsstoff(e).

4. Kit gemäß irgendeinem der Ansprüche 1 bis 3, umfassend oder bestehend aus mit Ramipril, dosiert zu 2 % (Gew./Vol) und Apraclonidin, dosiert zu 0,5 % (Gew./Vol.).

5. Kit gemäß irgendeinem der Ansprüche 1 bis 3, umfassend oder bestehend aus Ramipril, dosiert zu 2 % (Gew./Vol.) und Brimonidon, dosiert zu 0,2 % (Gew./Vol.).

6. Kit gemäß irgendeinem der Ansprüche 1 bis 3, umfassend oder bestehend aus Ramipril, dosiert zu 2 % (Gew./Vol.), Neosynephrin, dosiert zu 10 % (Gew./Vol.) und Timolol, dosiert zu 0,5 % (Gew./Vol.).

7. Ophthalmologische Zusammensetzung, umfassend oder bestehend aus den Zutaten eines Kits gemäß Definition in irgendeinem der Ansprüche 1 bis 6.

8. Kit oder ophthalmologische Zusammensetzung gemäß irgendeinem der Ansprüche 1 bis 7 zur Verwendung als ein Medikament.

9. Kit oder ophthalmologische Zusammensetzung zur Verwendung gemäß Anspruch 8 zur Behandlung von Störungen und/oder Krankheiten des Auges.

10. Kit oder ophthalmologische Zusammensetzung zur Verwendung gemäß irgendeinem der Ansprüche 8 oder 9 zur topischen Verabreichung.

11. Kit oder ophthalmologische Zusammensetzung zur Verwendung gemäß irgendeinem der Ansprüche 8 bis 10, wobei Apraclonidin, Brimonidin, Neosynephrin und/oder Timolol das Auge vor einer effektiven Menge an Ramipril kontaktiert (kontaktieren).

12. Kit oder ophthalmologische Zusammensetzung zur Verwendung gemäß irgendeinem der Ansprüche 8 bis 11 zur Ausgabe von Ramipril an das hintere Segment der Augen und insbesondere an die Chorioretina und die optische Papille eines Auges.

13. Kit oder ophthalmologische Zusammensetzung zur Verwendung gemäß irgendeinem der Ansprüche 8 bis 12 zur Behandlung wenigstens einer Augenstörung und/oder Krankheit des Auges, die aus der Gruppe Glaukom, glaukomatöse Neuropathie, diabetische Retinopathie, retinaler Venenverschluss, insbesondere zentraler retinaler Venenverschluss oder retinaler Venenverschluss eines Zweiges, makulöses Ödem, trockene und exsudative makulöse Degeneration, insbesondere altersbedingte makulöse Degeneration, ausgewählt ist.

14. Kit oder ophthalmologische Zusammensetzung zur Verwendung gemäß irgendeinem der Ansprüche 8 bis 13, wobei Apraclonidin, Brimonidin oder Neosynphrin einer Person oder einem Tier, die / das dies benötigt, in einer Konzentration verabreicht wird (werden), die von 0,01 % bis 20 % (Gew./Vol.), bevorzugt von 0,1 % bis 15 % (Gew./Vol.) und weiter bevorzugt von 0,1 bis 3 % (Gew./Vol.) reicht, Timolol einer Person oder einem Tier, die / das dies benötigt, in einer Konzentration verabreicht wird, die von 0,05 % bis 2 % (Gew./Vol.), bevorzugt von 0,1 % bis 1 % (Gew./Vol.) und weiter bevorzugt von 0,1 % bis 0,5 % (Gew./Vol.) reicht, Ramipril einer Person oder einem Tier, die / das dies benötigt, in einer Konzentration verabreicht wird, die von 0,001 % bis 15 % (Gew./Vol.), bevorzugt von 0,05 bis 10 % (Gew./Vol) und weiter bevorzugt von 0,1 % bis 3 % (Gew./Vol.) reicht.

## Revendications

1. Kit comprenant ou constitué de :
a) ramipril et apraclonidine, ou
b) ramipril et brimonidine, ou
c) ramipril, néosynéphrine, et timolol,
dans lequel l'apraclonidine, la brimonidine, la néosynéphrine et le timolol peuvent être administrés simultanément au ramipril ou avant celui-ci.

2. Kit selon la revendication 1, dans lequel l'apraclonidine, la brimonidine ou la néosynéphrine sont dosées en des quantités physiologiquement acceptables de 0,01 % à 20 % (p/v), de préférence de 0,1 % à 15 % (p/v), et de manière davantage préférée de 0,1 à 3 % (p/v), le timolol est dosé en une quantité physiologiquement acceptable de 0,05 % à 2 % (p/v), de préférence de 0,1 % à 1 % (p/v), et de manière davantage préférée de 0,1 à 0,5 % (p/v), et le ramipril est dosé en une quantité physiologiquement acceptable dans la plage de 0,001 % à 15 % (p/v), de préférence de 0,05 % à 10 % (p/v), et de manière davantage préférée de 0,1 à 3 % (p/v).

3. Kit selon l'une quelconque des revendications 1 et 2, comprenant en outre un vecteur acceptable sur le plan ophtalmologique, tel que l'eau, notamment l'eau distillée ou désionisée, une solution saline, notamment une solution saline tamponnée au phosphate, ou un (des) excipient(s).

4. Kit selon l'une quelconque des revendications 1 à 3, comprenant ou constitué de ramipril dosé à 2 % (p/v) et d'apraclonidine dosée à 0,5 % (p/v).

5. Kit selon l'une quelconque des revendications 1 à 3, comprenant ou constitué de ramipril dosé à 2 % (p/v) et de brimonidine dosée à 0,2 % (p/v).

6. Kit selon l'une quelconque des revendications 1 à 3, comprenant ou constitué de ramipril dosé à 2 % (p/v), de néosynéphrine dosée à 10 % (p/v), et de timolol dosé à 0,5 % (p/v).

7. Composition ophtalmologique comprenant ou constituée des ingrédients d'un kit tel que défini dans l'une quelconque des revendications 1 à 6.

8. Kit ou composition ophtalmologique selon l'une quelconque des revendications 1 à 7, pour son utilisation en tant que médicament.

9. Kit ou composition ophtalmologique pour son utilisation selon la revendication 8, pour le traitement de troubles et/ou de maladies des yeux.

10. Kit ou composition ophtalmologique pour son utilisation selon l'une quelconque des revendications 8 ou 9, pour une administration topique.

11. Kit ou composition ophtalmologique pour son utilisation selon l'une quelconque des revendications 8 à 10, dans lequel l'apraclonidine, la brimonidine, la néosynéphrine, et/ou le timolol entre (nt) en contact avec l'oeil avant une quantité efficace de ramipril.

12. Kit ou composition ophtalmologique pour son utilisation selon l'une quelconque des revendications 8 à 11, pour l'administration de ramipril au segment postérieur des yeux, et notamment à la choriorétine et à la tête du nerf optique d'un oeil.

13. Kit ou composition ophtalmologique pour son utilisation selon l'une quelconque des revendications 8 à 12, pour le traitement d'au moins un trouble oculaire et/ou une maladie de l'oeil sélectionnés dans le groupe constitué du glaucome, d'une neuropathie glaucomateuse, d'une rétinopathie diabétique, d'une occlusion de la veine rétinienne, notamment d'une occlusion de la veine centrale rétinienne ou d'une occlusion de la branche veineuse rétinienne, d'un oedème maculaire, de la dégénérescence maculaire sèche et humide, en particulier de la dégénérescence maculaire liée à l'âge.

14. Kit ou composition ophtalmologique pour son utilisation selon l'une quelconque des revendications 8 à 13, dans lequel l'apraclonidine, la brimonidine ou la néosynéphrine est (sont) administrées à un individu ou un animal le nécessitant en une concentration dans la plage de 0,01 % à 20 % (p/v), de préférence de 0,1 % à 15 % (p/v), et de manière davantage préférée de 0,1 à 3 % (p/v), le timolol est administré à un individu ou un animal le nécessitant en une concentration dans la plage de 0,05 % à 2 % (p/v), de préférence de 0,1 % à 1 % (p/v), et de manière davantage préférée de 0,1 à 0,5 % (p/v), le ramipril est administré à un individu ou un animal le nécessitant en une concentration dans la plage de 0,001 % à 15 % (p/v), de préférence de 0,05 % à 10 % (p/v), et de manière davantage préférée de 0,1 à 3 % (p/v).
